# EUROPEAN PATENT SPECIFICATION

(11) **EP 2 376 483 B1**
(45) Date of publication and mention of the grant of the patent: **20.03.2013**
(21) Application number: 09801190.1
(22) Date of filing: 09.12.2009
(51) Int. Cl.: C07D 409/12, A61K 31/4178, A61P 31/00, A01N 43/50

(54) **POLYMORPH II OF AN ANTIFUNGAL COMPOUND**
POLYMORPH II EINER VERBINDUNG MIT ANTIPILZWIRKUNG
POLYMORPHE II D UN COMPOSÉ ANTIFONGIQUE

(30) Priority: 09.12.2008 ES 200803473
(43) Date of publication of application: 19.10.2011
(73) Proprietor: Ferrer Internacional, S.A., 08028 Barcelona (ES)
(72) Inventor: DELGADO, Mónica, E-08018 Barcelona (ES); ALBET, Carles, E-08906 L'Hospitalet de Llobregat (ES); PETSCHEN OLIVÉ, Inés, E-08006 Barcelona (ES); TARRUELLA, Marta, E-25214 Santa Fe d'Olugues (ES)
(74) Representative: ZBM Patents
(86) International application number: PCT/EP2009/066663
(87) International publication number: WO 2010/066756

(56) References cited:
- WO-A1-03/068770

## Description

### Technical Field of the Invention

The invention relates to antifungal agents, specifically imidazole compounds. More particularly, the invention relates to a crystalline polymorph of a pharmaceutically acceptable salt of an optically active antifungal imidazole compound, the pharmaceutical and agricultural compositions containing such polymorph, its use in the treatment or prevention of skin or mucous membrane infections caused by fungi or yeasts in humans or pets, and its use in the treatment or prevention of agricultural diseases produced by such infectious agents.

### Background of the Invention

EP151477B1 discloses imidazole compounds of general formula **(I):** 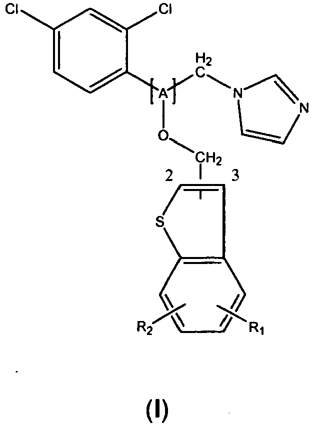 wherein [A], R₁ and R₂ have the meanings therein described, as well as pharmaceutical compositions containing them, and their use for treating fungal infections in humans and animals or for combating crop diseases. Compound (I) -specific when [A]=CH, R₁=H, R₂=7-Cl and the CH₂ group is bonded to the benzo[b]thiophene ring at the 3 position- is known as sertaconazole and is widely used in therapeutics as an antifungal agent, preferably in the form of mononitrate salt.

In turn, EP1474422B1 discloses the R-enantiomer of sertaconazole, i.e., R-(-)-1-[2-(7-chlorobenzo[*b*]thiophen-3-yl-methoxy)-2-(2,4-dichlorophenyl-ethyl]-1H-imidazole, of formula **(II,** arasertaconazole): 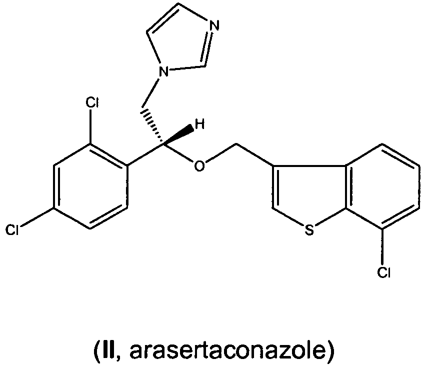 and its preferred salt, which is the mononitrate, of formula **(III,** arasertaconazole mononitrate): 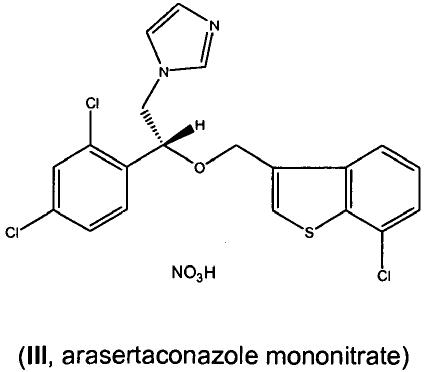 as well as its pharmaceutical and agricultural compositions, and its use in the preparation of pharmaceutical compositions for the treatment of infections caused by fungi and yeasts in humans and animals or agricultural compositions for the treatment of crop diseases produced by such microorganisms. Arasertaconazol mononitrate **(III)** optionally mixed with pharmaceutically acceptable carriers can be administered to humans or animals by the oral route in the form of tablets, capsules, coated tablets, syrups, solutions, powders, granules, emulsions, oral gels, oral pastes, buccopharingeal solutions, buccopharingeal suspensions, buccopharingeal gels, buccopharingeal pastes, etc., by injection, by rectal route and by vaginal-intrauterine route in the form of ovulum, vaginal tablet, vaginal capsule, medicated vaginal tampon, ointment, cream, gel, foam, solution, emulsion, suspension, pessary, lotion, etc., at daily doses ranging from 50 to 400 mg; and by topical route in the form of cream, lotion, paste, suspension, ointment, emulsion, solution, foam, shampoo, powder, gel, etc., at concentrations ranging from 0.05 to 3%. Also the compound of the invention can be optionally applied in admixture with a diluent or carrier against crop diseases by watering, atomizing, spraying, dusting, or in the form of powder, cream, paste, etc., at the rate of 0.05 - 10 Kg per hectare of soil.

EP1474422B1 assigns the compound **(III)** obtained in Example 4 with a DSC peak at 116.87°C. In the course of our present investigations, it was found out that there was a typing error in the above DSC peak, which should be corrected to read 171.6°C.

### Brief Description of the Drawings

Figures 1 and 2 show the Differential Scanning Calorimetry thermograms of compound **(III)** as polymorph I and polymorph II respectively. The ordinate represents the heat flow expressed in mW. The abscissa indicates the temperature expressed in °C.

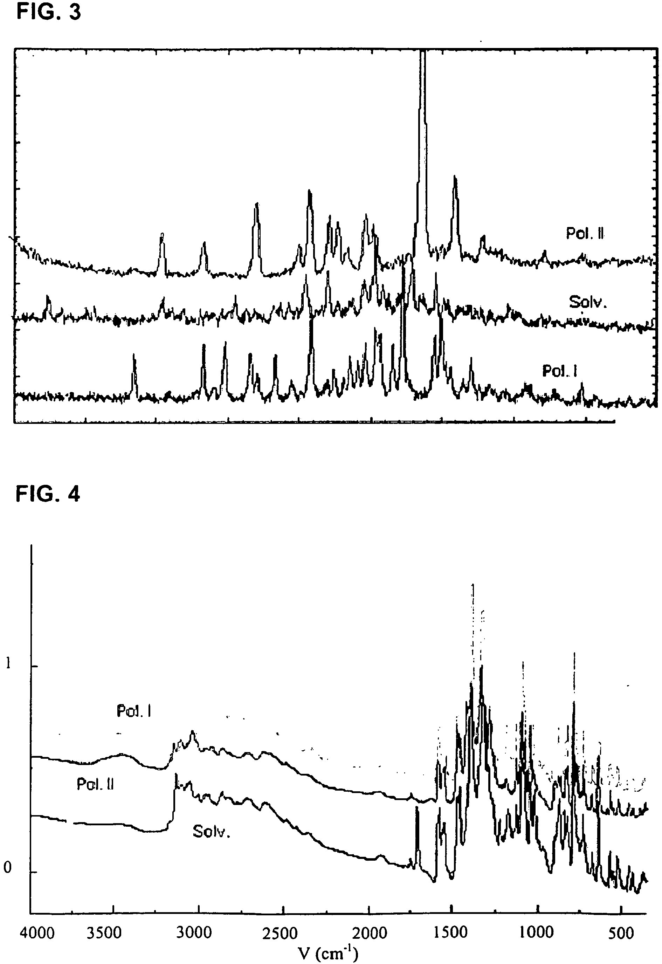

Figure 3 shows the X-ray Powder Diffraction curves of compound **(III)** as polymorph I, polymorph II and solvate with ½ mole of acetone **(IV).** The intensity, in ordinates, is expressed in cps. The abscissa corresponds to 2θ° angle.
Figure 4 shows the IR spectrum of compound **(III)** as polymorph I, polymorph II and solvate with ½ mole of acetone (IV).

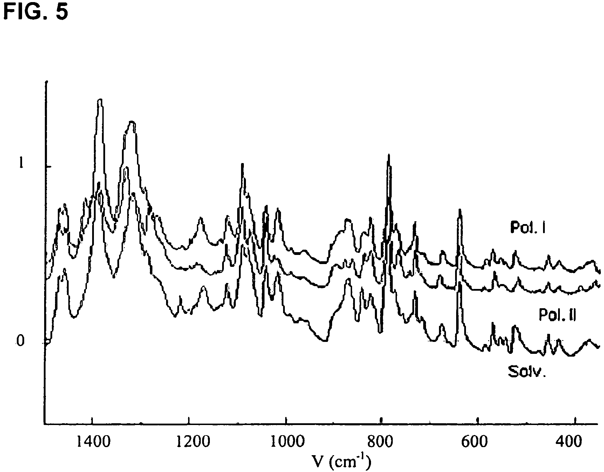

Figure 5 shows a zone selected from the IR spectrum of compound **(III)** as polymorph I, polymorph II and solvate with ½ mole of acetone **(IV).**

### Detailed Description of the Invention

The present invention refers to polymorph II of arasertaconazole mononitrate (III), as well as methods for its preparation, its use in pharmacy and agriculture and the pharmaceutical and agricultural compositions comprising the novel polymorph.

Polymorph II of arasertaconazole mononitrate **(III)** constitutes the main object of the present application. Polymorph II, in contrast to polymorph I, exhibits the outstanding property that its density is much lower than polymorph I. In fact, polymorph II presents an apparent density of 0.39g/mL and a compact density of 0.53g/mL, as compared to polymorph I whose apparent density is 0.78g/mL and whose compact density is 1.08g/mL.

The low density of the novel polymorph shows important advantages in both formulation processes at industrial scale and the proper nature of certain types of topical formulations, as in the case of foams, aerosols and solid powders. The advantages of polymorph II of arasertaconazole mononitrate **(III)** are described as follows.

### General advantages in formulation processes

A low density may help to dissolve or disperse an active ingredient more rapidly and, therefore, may be of interest in some stages of the formulation processes at industrial scale. In addition, surprisingly, polymorph II is more stable at high temperature, thereby polymorph I is preferred when required to operate at high temperatures (higher than 80°C).

### Advantages of some types of topical pharmaceutical formulations

### Foams

Foams have an apparent lower density than creams because air is incorporated. An elemental formulation principle recommends approaching the densities and sizes of the different particles in a slurry or solid emulsion in order to minimize the sedimentation effects over time and thus increase the stability of the final formulation.

### Aerosols and sprays

The apparent low density of aerosols usually involves a low energy fragmentation of the solid, thus facilitating the reduction in smaller particles that are to be atomized or sprayed.

### Solid powder

A large treatment area is available by using a less dense active ingredient.

Thus, in a first embodiment, the present application provides the novel polymorph II of arasertaconazole mononitrate **(III)** characterized by presenting:
(i) a melting point within the range of 170°-172°C;
(ii) the following crystallographic characteristics:

| | |
|---|---|
| Wavelength (Å) | 0.71069 |
| Crystal system, space group | Orthorhombic, P2₁2₁2₁ |

| Unit cell dimensions | |
|---|---|
| A (Å) | 9.3550(10) |
| B (Å) | 9.5010(10) |
| C (Å) | 25.3810(10) |
| Volume (Å³) | 2255.9(3) |
| Z, Density (calculated) | 4, 1.474 mg/m³ |
| µ (mm⁻¹) | 0.531 |
| F(000) | 1024 |
| Crystal size (mm) | 0.1 x 0.2 x 0.2 |
| θ Range | 1.60 - 25.00 degrees |
| Index ranges | 0≤h≤11, 0≤k≤10, 0≤I≤30 |
| Reflections collected / Independent reflections | 5610 / 1976 [R(int) = 0.10] |
| Integrity at 2θ | 85.5% |
| Data / parameters | 1976 / 283 |
| Goodness-of-Fit F² | 0.912 |
| Final R indices [I>2sigma(I)] | R1 = 0.048, wR2 = 0.104 |
| ÇR indices (all data) | R1 = 0.141, wR2 = 0.136 |
| Absolute structure parameter | -0.11(16) |
| Largest difference peak and hole | 0.241 and -0.136 e.A⁻³ |

and
(iii) the following X-ray diffraction peak intensities and spacing:

| **h** | **k** | **I** | **D** | **I** | **h** | **k** | **I** | **d** | **I** | **h** | **k** | **I** | **d** | **I** |
|---|---|---|---|---|---|---|---|---|---|---|---|---|---|---|
| 0 | 1 | 1 | 8.8980 | 74. | 0 | 1 | 8 | 3.0093 | 27. | 1 | 1 | 11 | 2.1804 | 22. |
| 1 | 0 | 1 | 8.7777 | 40. | 1 | 3 | 0 | 2.9998 | 30. | 1 | 3 | 8 | 2.1797 | 20. |
| 0 | 1 | 2 | 7.6057 | 280. | 2 | 1 | 6 | 2.9792 | 45. | 1 | 2 | 10 | 2.1772 | 11. |
| 1 | 1 | 0 | 6.6660 | 14. | 1 | 3 | 1 | 2.9790 | 9. | 2 | 2 | 9 | 2.1529 | 10. |
| 1 | 1 | 1 | 6.4473 | 183. | 0 | 3 | 3 | 2.9660 | 26. | 0 | 4 | 5 | 2.1514 | 15. |
| 0 | 0 | 4 | 6.3453 | 85. | 2 | 2 | 4 | 2.9507 | 31. | 3 | 3 | 3 | 2.1491 | 9. |
| 0 | 1 | 3 | 6.3184 | 40. | 3 | 1 | 1 | 2.9428 | 141. | 3 | 2 | 7 | 2.1166 | 24. |
| 1 | 0 | 3 | 6.2749 | 10. | 1 | 3 | 2 | 2.9193 | 106. | 0 | 3 | 9 | 2.1061 | 18. |
| 1 | 1 | 2 | 5.9014 | 58. | 3 | 1 | 2 | 2.8852 | 41. | 1 | 4 | 5 | 2.0966 | 18. |
| 0 | 1 | 4 | 5.2767 | 100. | 0 | 2 | 7 | 2.8822 | 10. | 4 | 2 | 1 | 2.0911 | 26. |
| 1 | 0 | 4 | 5.2513 | 28. | 1 | 1 | 8 | 2.8647 | 10. | 4 | 2 | 2 | 2.0701 | 42. |
| 1 | 1 | 3 | 5.2360 | 636. | 0 | 3 | 4 | 2.8337 | 18. | 1 | 0 | 12 | 2.0630 | 8. |
| 0 | 2 | 0 | 4.7505 | 41. | 2 | 2 | 5 | 2.7861 | 23. | 1 | 3 | 9 | 2.0547 | 13. |
| 0 | 2 | 1 | 4.6694 | 16. | 2 | 1 | 7 | 2.7436 | 8. | 3 | 1 | 9 | 2.0427 | 32. |
| 2 | 0 | 1 | 4.6000 | 66. | 1 | 3 | 4 | 2.7120 | 37. | 4 | 2 | 3 | 2.0366 | 11. |
| 1 | 1 | 4 | 4.5960 | 225. | 0 | 3 | 5 | 2.6869 | 10. | 3 | 3 | 5 | 2.0355 | 20. |
| 0 | 1 | 5 | 4.4772 | 83. | 2 | 2 | 6 | 2.6180 | 10. | 1 | 2 | 11 | 2.0262 | 18. |
| 1 | 0 | 5 | 4.4617 | 160. | 2 | 3 | 1 | 2.6086 | 19. | 2 | 1 | 11 | 2.0219 | 36. |
| 0 | 2 | 2 | 4.4490 | 382. | 3 | 2 | 0 | 2.6069 | 10. | 1 | 1 | 12 | 2.0160 | 10. |
| 1 | 2 | 0 | 4.2357 | 21. | 1 | 1 | 9 | 2.5972 | 14. | 3 | 2 | 8 | 2.0142 | 27. |
| 0 | 0 | 6 | 4.2302 | 89. | 3 | 1 | 5 | 2.5589 | 11. | 2 | 4 | 4 | 2.0089 | 20. |
| 0 | 2 | 3 | 4.1422 | 312. | 3 | 2 | 2 | 2.5536 | 23. | 4 | 1 | 6 | 2.0009 | 10. |
| 2 | 1 | 1 | 4.1403 | 21. | 0 | 0 | 10 | 2.5381 | 9. | 0 | 3 | 10 | 1.9806 | 9. |
| 2 | 0 | 3 | 4.0935 | 18. | 0 | 3 | 6 | 2.5352 | 9. | 2 | 4 | 5 | 1.9545 | 8. |
| 1 | 1 | 5 | 4.0385 | 175. | 2 | 1 | 8 | 2.5308 | 9. | 3 | 1 | 10 | 1.9275 | 10. |
| 2 | 1 | 2 | 3.9843 | 44. | 2 | 3 | 3 | 2.5049 | 9. | 2 | 2 | 11 | 1.8971 | 17. |
| 0 | 1 | 6 | 3.8644 | 23. | 1 | 3 | 6 | 2.4470 | 10. | 4 | 3 | 1 | 1.8762 | 15. |
| 1 | 0 | 6 | 3.8544 | 171. | 2 | 3 | 4 | 2.4236 | 8. | 1 | 1 | 13 | 1.8737 | 12. |
| 0 | 2 | 4 | 3.8028 | 107. | 0 | 4 | 1 | 2.3649 | 12. | 3 | 4 | 2 | 1.8689 | 13. |
| 1 | 2 | 3 | 3.7875 | 45. | 3 | 0 | 7 | 2.3642 | 9. | 0 | 3 | 11 | 1.8649 | 9. |
| 2 | 0 | 4 | 3.7651 | 315. | 1 | 2 | 9 | 2.3474 | 12. | 1 | 5 | 2 | 1.8424 | 17. |
| 2 | 1 | 3 | 3.7594 | 176. | 2 | 3 | 5 | 2.3299 | 26. | 2 | 4 | 7 | 1.8287 | 10. |
| 1 | 1 | 6 | 3.5717 | 47. | 4 | 0 | 1 | 2.3289 | 10. | 5 | 1 | 2 | 1.8168 | 9. |
| 1 | 2 | 4 | 3.5229 | 31. | 3 | 2 | 5 | 2.3190 | 8. | 0 | 0 | 14 | 1.8129 | 31. |
| 2 | 1 | 4 | 3.5002 | 128. | 1 | 3 | 7 | 2.3113 | 10. | 5 | 1 | 3 | 1.7940 | 10. |
| 2 | 0 | 5 | 3.4398 | 34. | 3 | 1 | 7 | 2.2943 | 33. | 2 | 2 | 12 | 1.7858 | 9. |
| 0 | 1 | 7 | 3.3876 | 1000. | 1 | 4 | 1 | 2.2928 | 10. | 1 | 4 | 9 | 1.7834 | 16. |
| 1 | 0 | 7 | 3.3808 | 25. | 4 | 1 | 1 | 2.2619 | 12. | 4 | 3 | 5 | 1.7641 | 14. |
| 2 | 2 | 1 | 3.3046 | 159. | 0 | 3 | 8 | 2.2414 | 14. | 4 | 2 | 8 | 1.7501 | 9. |
| 1 | 2 | 5 | 3.2522 | 54. | 0 | 2 | 10 | 2.2386 | 9. | 1 | 1 | 14 | 1.7494 | 17. |
| 2 | 1 | 5 | 3.2344 | 10. | 2 | 0 | 10 | 2.2308 | 14. | 1 | 3 | 12 | 1.7286 | 20. |
| 2 | 2 | 2 | 3.2237 | 54. | 2 | 3 | 6 | 2.2289 | 10. | 2 | 5 | 3 | 1.7236 | 9. |
| 1 | 1 | 7 | 3.1852 | 32. | 3 | 3 | 0 | 2.2220 | 26. | 2 | 2 | 13 | 1.6846 | 10. |
| 0 | 0 | 8 | 3.1726 | 162. | 3 | 2 | 6 | 2.2193 | 36. | 5 | 1 | 6 | 1.6840 | 13 |
| 0 | 2 | 6 | 3.1592 | 77. | 3 | 3 | 1 | 2.2135 | 10. | 0 | 1 | 15 | 1.6659 | 12. |
| 2 | 2 | 3 | 3.1010 | 119. | 4 | 0 | 4 | 2.1944 | 29. | 3 | 5 | 2 | 1.6096 | 8. |
| 0 | 3 | 2 | 3.0728 | 39. | 3 | 3 | 2 | 2.1887 | 11. | 1 | 6 | 1 | 1.5583 | 9. |

Moreover, the new polymorph is characterized by presenting a DSC (Differential Scanning Calorimetry) endothermal peak at 171.5°C

In another embodiment, the present invention provides a process for the preparation of the new polymorph comprising heating of the solvated form of arasertaconazole mononitrate with ½ mole of acetone, of formula **(IV):** 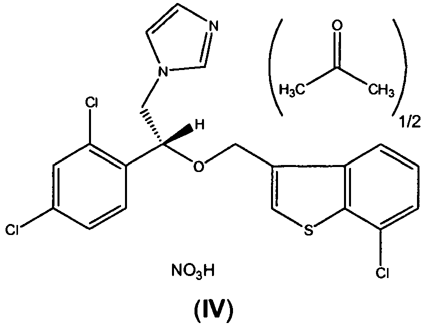 at a temperature of 354.2°K.

In another embodiment, the present invention provides a process for the preparation of the new polymorph comprising heating of polymorph I at 373°K over a period from 2 to 24h.

In another embodiment, the present invention provides a process for the preparation of the new polymorph comprising crystallization of polymorph I or the solvated form of arasertaconazole mononitrate with ½ mole of acetone **(IV)** in water.

In another embodiment, the present invention provides the use of the new polymorph for the preparation of pharmaceutical compositions for the treatment or prevention of skin or mucous membrane infections caused by fungi or yeasts in humans or pets. This aspect can also be formulated as a method of treating or preventing skin or mucous membrane infections caused by fungi or yeasts in humans or pets, comprising the administration to said human or pet in need thereof of a therapeutically effective amount of the polymorph as defined in the present invention.

The term "therapeutically effective amount" refers to the amount of the polymorph of the invention that, when administered, is sufficient to prevent development of, or alleviate to some extent, one or more of the symptoms of the infection. The term "therapeutically effective amount" also refers to the amount of polymorph of the invention that is sufficient to elicit the biological or medical response of a cell, tissue, system, animal or human that is being sought by a researcher, veterinarian, medical, doctor or clinician.

In another embodiment, the present invention provides the use of the new polymorph for the preparation of agricultural compositions for the treatment or prevention of crop diseases produced by fungi and yeasts.

In another embodiment, the present invention provides pharmaceutical compositions comprising the new polymorph and pharmaceutically acceptable carriers for the treatment or prevention of skin or mucous membrane infections caused by fungi or yeasts in humans or pets.

In another embodiment, the present invention provides agricultural compositions comprising the new polymorph and agriculturally acceptable carriers for the treatment or prevention of crop diseases produced by fungi and yeasts.

### Particular Embodiments of the Invention

The crystallographic studies of the present invention were carried out in accordance with the following specifications.

### Instruments

### Microbalance (Mettler, MT5)

### Analytical balance (Mettler, AT261)

Differential Scanning Calorimeter, DSC (Mettler, DSC820C)
- Calibration and titration of substances (Mettler, ME-119422)
- 40-µl Al Crucible without pin (Mettler, ME-26763)

Thermogravimetric analyzer (Mettler-Toledo, TG50 / MT5, TA-8000)
- 70-µl Al₂O₃ Crucible with perforated lid

IR Spectrophotometer (Thermo Nicolet, Nexus) equipped with a DTGS KBr detector. Software: Advanced OMNIC CD, v 6.0a for Nexus, together with all of the necessary accessories needed for the preparation of solid samples that are dispersed in 13mm KBr tablets.

### Microscope (Nikon, Eclipse E-600)

- Lens: 4x, 10x, 20x and 40x.
- Filter and polarized light analyzer.
- Video camera (Sony, SSC-C370P)
- Software for Windows (Linkam, LinkSys-310RTVMS,), software for real time video digitalization with calibrated lens.

### Experimental conditions

### Differential Scanning Calorimetry (DSC)

### - Sample preparation

About 3mg of the test substances were carefully weighed into the 40-µl aluminum crucible. The lid was perforated and hermetically sealed, the crucible was sealed off, and the lid was equalized. Then, the crucible was placed into the furnace and the experiment started.

### - Data acquisition and processing techniques

The samples were heated from 30 to 200°C at a rate of 10°C/min under a N₂ stream, the flow rate being 80mL/min.

### - Thermogravimetry (TG)

### - Sample preparation

The necessary amounts were carefully weighed for completing ¾ parts of the 70-µl Al₂O₃ crucible provided with a perforated lid that was hermetically sealed. Then, the crucible was placed into the furnace and the experiment started.

### - Data acquisition and processing techniques

The samples were heated from 30 to 120°C at a rate of 5°C/min under a N₂ stream, the flow rate being 80mL/min. Mass loss rate was calculated in relation to baseline by horizontal tangent plot.

### - Infrared spectroscopy (IR)

### - Sample preparation

About 1 mg of test substance and 200mg of potassium bromide were ground in an agate mortar. The ground and homogenized mixture was then transferred to a 13mm diameter die under a pressure of about 800 MP thus giving a homogenous tablet. The die was withdrawn, the tablet was placed on the tablet holder using a clamp, and the tablet was fitted into the apparatus compartment. Then, spectrum measurement was performed.

### - Data acquisition and processing techniques

Resolution: 4 cm⁻¹
Spectral range (x-frequency): 4000 cm⁻¹ at 400 cm⁻¹
Laser range: 15798.3 cm⁻¹
Final polymorph (y): % Transmittance
Number of spectrum accumulations: 32
Number of historical accumulations: 32
Historical acquisition: automatic mode prior to each spectrum and final suppression.
Apodization: Happ-Genzel
Phase correction: Mertz
Baseline correction: automatic

### Polymorphism study

Full observation of spectra showed that the same functional groups are present in both polymorphs while referring to the same molecule. A more detailed comparison of spectra revealed small changes in position and intensity in the bands of the three specific spectral regions that were chosen for comparison by the searching software. The three spectral regions were as follows:
Region 1: Range from 3175 cm⁻¹ to 3000 cm⁻¹
Region 2: Range from 1360 cm⁻¹ to 1150 cm⁻¹
Region 3: Range from 540 cm⁻¹ to 510 cm⁻¹

### Example 1: Polymorph I of arasertaconazole mononitrate (III)

A solution of arasertaconazole mononitrate **(III),** previously prepared according to EP1474422B1 (Example 4) in any of the solvents selected from heptane, methanol, ethanol, toluene, 4-chlorotoluene, acetic acid, ethyl acetate, trichloromethane, or a combination thereof with water (50:50, v/v), was verified to provide by addition of water a crystalline precipitate mostly containing the product under the same crystallographic characteristics as the starting material; however, polymorph I was obtained only in a pure form when mixtures of ethanol/water and ethyl acetate/water were used (Table 1).

**Table 1. Crystal data and structure refinements for polymorph I of arasertaconazole mononitrate (III)**

| | |
|---|---|
| Molecular formula | C₂₀H₁₆Cl₃N₃O₄S |
| Molecular weight | 500.77 |
| Wavelength (Å) | 0.71069 |
| Crystal system, space group | Monoclinic, P2₁ |

| Unit cell dimensions | |
|---|---|
| A (Å) | 9.2710(10) |
| B (Å) | 9.3160(10) |
| C (Å) | 13.3360(10) |
| β (°) = | 103.3380(10) |
| Volume (Å³) | 1120.74(19) |
| Z, Density (calculated) | 2, 1.484 mg/m³ |
| µ (mm⁻¹) | 0.534 |
| F(000) | 512 |
| Crystal size (mm) | 0.1 x 0.1 x 0.2 |
| Θ Range | 2.26 - 31.60 degrees |
| Index range | 0≤h≤13, 0≤k≤12, -19≤I≤19 |
| Reflections collected / Independent reflections | 3478 / 3477 [R(int) = 0.01] |
| Integrity at 2θ | 87.4% |
| Data / parameters | 3477 / 353 |
| Goodness-of-Fit F² | 1.103 |
| Final R indices [I>2sigma(I)] | R1 = 0.064, wR2 = 0.153 |
| R indices (all data) | R1 = 0.119, wR2 = 0.176 |
| Absolute structure parameter | -0.02(10) |
| Extinction coefficient | 0.010(5) |
| Largest difference peak and hole | 0.216 and -0.225 e.A⁻³ |

### Differential Scanning Calorimetry (DSC)

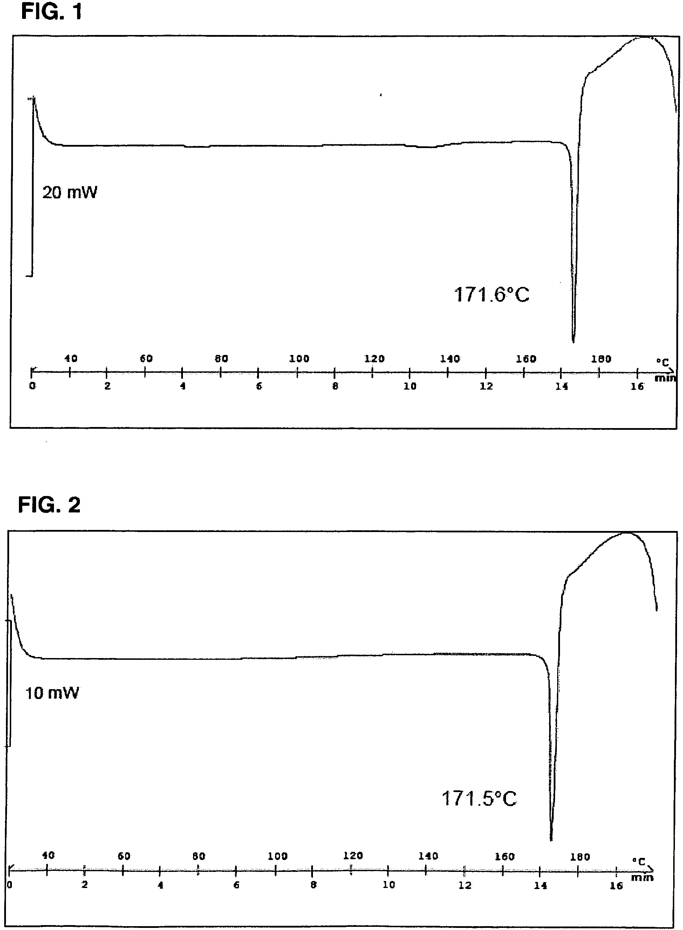

The main peak was detected at 171.6°C, i.e., the characteristic melting peak within the range of 170°-172°C, Figure 1.

**Table 2. X-ray diffraction peak intensities and spacing for polymorph I of arasertaconazole mononitrate (III)**

| **h** | **k** | **I** | **D** | **I** | **h** | **k** | **I** | **d** | **I** | **h** | **k** | **I** | **d** | **I** |
|---|---|---|---|---|---|---|---|---|---|---|---|---|---|---|
| 1 | 0 | 0 | 9.0209 | 378. | 0 | 2 | 4 | 2.6621 | 13. | 3 | 3 | 2 | 1.9548 | 24. |
| 0 | 1 | 1 | 7.5677 | 41. | 1 | 3 | 2 | 2.6026 | 30. | 2 | 2 | -6 | 1.9546 | 26. |
| 1 | 0 | 1 | 6.7163 | 21. | 0 | 0 | 5 | 2.5953 | 10. | 2 | 3 | -5 | 1.9540 | 44. |
| 0 | 0 | 2 | 6.4881 | 140. | 3 | 2 | -1 | 2.5751 | 9. | 2 | 4 | -3 | 1.9488 | 29. |
| 1 | 1 | 0 | 6.4806 | 307. | 2 | 3 | -1 | 2.5742 | 38. | 3 | 1 | 4 | 1.9446 | 13. |
| 1 | 1 | -1 | 6.2248 | 57. | 2 | 3 | 0 | 2.5578 | 17. | 4 | 0 | -5 | 1.9380 | 18. |
| 1 | 0 | -2 | 5.9591 | 485. | 3 | 2 | -2 | 2.5254 | 51. | 2 | 4 | 2 | 1.9138 | 13. |
| 1 | 1 | 1 | 5.4481 | 552. | 0 | 3 | 3 | 2.5226 | 24. | 4 | 2 | -4 | 1.9081 | 12. |
| 0 | 1 | 2 | 5.3242 | 186. | 2 | 0 | -5 | 2.5141 | 22. | 1 | 0 | -7 | 1.9045 | 15. |
| 1 | 1 | -2 | 5.0199 | 307. | 3 | 0 | -4 | 2.5132 | 37. | 0 | 4 | 4 | 1.8919 | 9. |
| 1 | 0 | 2 | 4.7713 | 172. | 0 | 1 | 5 | 2.5001 | 8. | 2 | 3 | 4 | 1.8918 | 14. |
| 2 | 0 | -1 | 4.6024 | 12. | 2 | 3 | -2 | 2.4934 | 25. | 1 | 3 | 5 | 1.8759 | 36. |
| 2 | 0 | 0 | 4.5105 | 796. | 2 | 3 | 1 | 2.4494 | 44. | 0 | 0 | 7 | 1.8538 | 26. |
| 0 | 2 | 1 | 4.3841 | 56. | 1 | 2 | 4 | 2.4313 | 11. | 5 | 0 | -1 | 1.8460 | 9. |
| 0 | 0 | 3 | 4.3254 | 76. | 2 | 1 | -5 | 2.4273 | 23. | 0 | 5 | 1 | 1.8443 | 21. |
| 1 | 0 | -3 | 4.3068 | 17. | 3 | 1 | -4 | 2.4265 | 11. | 3 | 4 | 0 | 1.8413 | 11. |
| 1 | 1 | 2 | 4.2467 | 279. | 3 | 2 | 1 | 2.3942 | 30. | 3 | 4 | -2 | 1.8410 | 22. |
| 2 | 1 | -1 | 4.1263 | 212. | 3 | 2 | -3 | 2.3928 | 18. | 1 | 2 | 6 | 1.8386 | 12. |
| 1 | 2 | -1 | 4.0698 | 73. | 2 | 0 | 4 | 2.3856 | 32. | 2 | 4 | -4 | 1.8349 | 17. |
| 2 | 1 | 0 | 4.0597 | 280. | 1 | 3 | 3 | 2.3488 | 15. | 4 | 2 | 2 | 1.8319 | 17. |
| 2 | 0 | 1 | 3.9849 | 188. | 2 | 3 | -3 | 2.3398 | 86. | 3 | 2 | 4 | 1.8288 | 13. |
| 0 | 1 | 3 | 3.9232 | 303. | 2 | 1 | 4 | 2.3111 | 22. | 3 | 2 | -6 | 1.8272 | 18. |
| 1 | 1 | -3 | 3.9092 | 235. | 0 | 4 | 1 | 2.2924 | 13. | 1 | 5 | 0 | 1.8247 | 21. |
| 1 | 2 | 1 | 3.8276 | 231. | 0 | 2 | 5 | 2.2671 | 10. | 0 | 1 | 7 | 1.8181 | 12. |
| 2 | 1 | -2 | 3.8162 | 265. | 1 | 3 | -4 | 2.2631 | 16. | 5 | 1 | -2 | 1.8165 | 9. |
| 0 | 2 | 2 | 3.7838 | 772. | 4 | 1 | -1 | 2.2456 | 18. | 4 | 3 | -3 | 1.8064 | 10. |
| 1 | 2 | -2 | 3.6699 | 177. | 1 | 4 | -1 | 2.2437 | 26. | 4 | 1 | 3 | 1.7993 | 10. |
| 2 | 1 | 1 | 3.6638 | 136. | 0 | 3 | 4 | 2.2432 | 16. | 4 | 2 | -5 | 1.7893 | 12. |
| 1 | 0 | 3 | 3.5907 | 1000. | 3 | 0 | 3 | 2.2388 | 20. | 3 | 4 | 1 | 1.7882 | 10. |
| 2 | 0 | -3 | 3.5589 | 61. | 4 | 1 | -2 | 2.2341 | 29. | 3 | 4 | -3 | 1.7876 | 9. |
| 2 | 0 | 2 | 3.3581 | 137. | 4 | 0 | -3 | 2.2208 | 19. | 5 | 1 | -3 | 1.7873 | 25. |
| 1 | 1 | 3 | 3.3504 | 379. | 1 | 4 | 1 | 2.2005 | 13. | 0 | 3 | 6 | 1.7747 | 30. |
| 1 | 2 | 2 | 3.3330 | 62. | 0 | 4 | 2 | 2.1920 | 37. | 5 | 1 | 0 | 1.7713 | 27. |
| 1 | 0 | -4 | 3.3051 | 479. | 3 | 3 | -1 | 2.1905 | 21. | 3 | 0 | -7 | 1.7710 | 10. |
| 2 | 2 | -1 | 3.2739 | 127. | 3 | 1 | 3 | 2.1768 | 77. | 2 | 3 | -6 | 1.7696 | 20. |
| 0 | 0 | 4 | 3.2441 | 307. | 3 | 1 | -5 | 2.1746 | 32. | 2 | 1 | 6 | 1.7629 | 22. |
| 0 | 2 | 3 | 3.1696 | 31. | 0 | 0 | 6 | 2.1627 | 8. | 3 | 1 | -7 | 1.7399 | 13. |
| 1 | 2 | -3 | 3.1622 | 15. | 1 | 1 | -6 | 2.1619 | 45. | 2 | 2 | -7 | 1.7380 | 9. |
| 2 | 1 | 2 | 3.1592 | 129. | 4 | 1 | -3 | 2.1602 | 14. | 0 | 4 | 5 | 1.7334 | 10. |
| 1 | 1 | -4 | 3.1149 | 191. | 3 | 3 | -2 | 2.1597 | 22. | 2 | 5 | -1 | 1.7270 | 18. |
| 2 | 2 | -2 | 3.1124 | 253. | 2 | 3 | -4 | 2.1499 | 11. | 2 | 5 | 0 | 1.7221 | 9. |
| 3 | 0 | -1 | 3.0903 | 60. | 4 | 0 | 1 | 2.1402 | 25. | 5 | 2 | -2 | 1.7210 | 10. |
| 0 | 1 | 4 | 3.0636 | 111. | 0 | 1 | 6 | 2.1067 | 17. | 2 | 3 | 5 | 1.7131 | 10. |
| 0 | 3 | 1 | 3.0201 | 76. | 1 | 3 | 4 | 2.0999 | 10. | 0 | 5 | 3 | 1.7112 | 17. |
| 3 | 0 | 0 | 3.0070 | 49. | 1 | 4 | 2 | 2.0930 | 21. | 1 | 1 | 7 | 1.7090 | 18. |
| 3 | 0 | -2 | 3.0055 | 44. | 4 | 0 | -4 | 2.0916 | 12. | 2 | 4 | -5 | 1.7085 | 10. |
| 2 | 0 | -4 | 2.9795 | 20. | 4 | 1 | 1 | 2.0859 | 28. | 3 | 4 | -4 | 1.7083 | 12. |
| 1 | 3 | 0 | 2.9362 | 59. | 2 | 4 | -1 | 2.0781 | 10. | 1 | 0 | -8 | 1.6647 | 12. |
| 3 | 1 | -1 | 2.9332 | 40. | 3 | 3 | 1 | 2.0759 | 43. | 3 | 2 | 5 | 1.6568 | 9. |
| 1 | 3 | -1 | 2.9113 | 56. | 3 | 3 | -3 | 2.0749 | 32. | 4 | 1 | 4 | 1.6524 | 22. |
| 3 | 1 | 0 | 2.8616 | 12. | 4 | 2 | -1 | 2.0722 | 21. | 1 | 1 | -8 | 1.6388 | 37. |
| 3 | 1 | -2 | 2.8604 | 22. | 2 | 4 | 0 | 2.0694 | 30. | 4 | 4 | -2 | 1.6369 | 8. |
| 1 | 0 | 4 | 2.8505 | 25. | 4 | 2 | -2 | 2.0632 | 11. | 4 | 4 | 0 | 1.6201 | 16. |
| 1 | 2 | 3 | 2.8438 | 50. | 1 | 4 | -3 | 2.0486 | 15. | 5 | 1 | 2 | 1.6182 | 11. |
| 2 | 1 | -4 | 2.8379 | 8. | 4 | 1 | -4 | 2.0408 | 21. | 4 | 4 | -3 | 1.6072 | 19. |
| 2 | 2 | -3 | 2.8279 | 55. | 2 | 4 | -2 | 2.0349 | 35. | 3 | 5 | -1 | 1.5956 | 18. |
| 1 | 3 | 1 | 2.8186 | 90. | 4 | 2 | 0 | 2.0298 | 41. | 3 | 1 | 6 | 1.5677 | 14. |
| 2 | 0 | 3 | 2.8144 | 37. | 1 | 3 | -5 | 2.0213 | 9. | 1 | 2 | -8 | 1.5676 | 9. |
| 0 | 3 | 2 | 2.8010 | 65. | 3 | 2 | 3 | 2.0178 | 25. | 2 | 5 | 3 | 1.5536 | 13. |
| 3 | 0 | 1 | 2.7912 | 16. | 3 | 2 | -5 | 2.0161 | 19. | 0 | 6 | 0 | 1.5527 | 9. |
| 3 | 0 | -3 | 2.7889 | 12. | 2 | 4 | 1 | 2.0108 | 26. | 2 | 4 | 5 | 1.5405 | 13. |
| 1 | 3 | -2 | 2.7539 | 36. | 1 | 2 | -6 | 2.0059 | 50. | 3 | 3 | 5 | 1.5396 | 8. |
| 1 | 1 | 4 | 2.7257 | 8. | 2 | 1 | 5 | 2.0058 | 33. | 3 | 3 | -7 | 1.5384 | 9. |
| 2 | 2 | 2 | 2.7240 | 78. | 4 | 2 | -3 | 2.0046 | 8. | 6 | 1 | -2 | 1.5243 | 8. |
| 2 | 1 | 3 | 2.6941 | 73. | 1 | 0 | 6 | 2.0011 | 22. | 4 | 4 | 2 | 1.5140 | 12. |
| 3 | 1 | 1 | 2.6737 | 72. | 0 | 3 | 5 | 1.9914 | 23. | | | | | |
| 3 | 1 | -3 | 2.6717 | 63. | 1 | 1 | 6 | 1.9564 | 23. | | | | | |

### Example 2: Solvated form of arasertaconazole mononitrate with ½ mole of acetone (IV)

The precipitation or crystallization of polymorph I of sertaconazole mononitrate (III) at a temperature below 333°K in acetone or any mixture (50:50, v/v) of acetone with other solvent, under a high vaporization temperature, such as methanol, ethanol, toluene, 4-chlorotoluene, acetic acid, ethyl acetate, trichloromethane, water, or binary mixture thereof, led to the solvated form of arasertaconazole mononitrate with ½ mole of acetone **(IV).** Crystallographic data of the obtained product are presented in Tables 3 and 4.

**Table 3. Crystal data and structure refinements for the solvated form of arasertaconazole mononitrate with ½ mole of acetone (IV)**

| | |
|---|---|
| Molecular formula | C₂₀H₁₆Cl₃N₃O₄S . 0.5 C₃H₆O |
| Molecular weight | 510.67 |
| Wavelength (Å) | 0.71069 |
| Crystal system, space group | Monoclinic, P2₁ |

| Unit cell dimensions | |
|---|---|
| A (Å) | 15.3710(10) |
| B (Å) | 9.7800(10) |
| C (Å) | 17.5570(10) |
| β (°) = | 92.7800(10) |
| Volume (Å³) | 2636.2(4) |
| Z, Density (calculated) | 4, 1.287 mg/m³ |
| µ (mm⁻¹) | 0.534 |
| F(000) | 512 |
| Crystal size (mm) | 0.1 x 0.1 x 0.2 |
| θ Range | 1.3 - 28.5 deg. |
| Index Range | 0≤h≤18, 0≤k≤12, -22≤I≤21 |
| Reflections collected / Independent reflections | 5644/ 5033 [R(int) = 0.03] |
| Integrity at 2θ | 87.4% |
| Data / parameters | 5033 / 596 |
| Goodness-of-Fit F² | 1.98 |
| Final R indices [I>2sigma(I)] | R1 = 0.1001, wR2 = 0.301 |
| Absolute structure parameter | -0.02(10) |
| Largest difference peak and hole | 0.38 and -0.47 e.A⁻³ |

**Table 4. X-ray diffraction peak intensities and spacing for the solvated form of arasertaconazole mononitrate with ½ mole of acetone (IV)**

| **h** | **k** | **I** | **D** | **I** | **H** | **k** | **I** | **D** | **I** | **h** | **k** | **I** | **d** | **I** |
|---|---|---|---|---|---|---|---|---|---|---|---|---|---|---|
| 1 | 0 | 0 | 15.3529 | 101. | 5 | 0 | 0 | 3.0706 | 109. | 3 | 1 | -7 | 2.2338 | 19. |
| 0 | 0 | 2 | 8.7682 | 265. | 5 | 0 | -1 | 3.0498 | 12. | 0 | 2 | 7 | 2.2296 | 14. |
| 0 | 1 | 1 | 8.5415 | 34. | 1 | 3 | -2 | 3.0066 | 17. | 6 | 2 | -2 | 2.2182 | 15. |
| 1 | 0 | -2 | 7.7781 | 69. | 5 | 0 | 1 | 2.9999 | 28. | 4 | 1 | 6 | 2.2138 | 9. |
| 1 | 1 | -1 | 7.5402 | 81. | 4 | 1 | 3 | 2.9891 | 34. | 3 | 0 | 7 | 2.2082 | 9. |
| 1 | 0 | 2 | 7.4597 | 76. | 1 | 3 | 2 | 2.9872 | 16. | 3 | 4 | 0 | 2.2061 | 17. |
| 1 | 1 | 1 | 7.3903 | 150. | 3 | 2 | 3 | 2.9813 | 90. | 6 | 1 | -4 | 2.2002 | 26. |
| 2 | 0 | -1 | 7.1610 | 9. | 2 | 2 | 4 | 2.9656 | 15. | 5 | 2 | 4 | 2.1973 | 23. |
| 2 | 0 | 1 | 6.9102 | 40. | 3 | 0 | -5 | 2.9608 | 18. | 7 | 0 | 0 | 2.1933 | 17. |
| 0 | 1 | 2 | 6.5285 | 611. | 5 | 0 | -2 | 2.9429 | 47. | 0 | 0 | 8 | 2.1920 | 20. |
| 1 | 1 | -2 | 6.0876 | 23. | 5 | 1 | 0 | 2.9296 | 14. | 0 | 3 | 6 | 2.1762 | 14. |
| 2 | 1 | 0 | 6.0385 | 115. | 1 | 0 | -6 | 2.8971 | 58. | 6 | 2 | 2 | 2.1725 | 8. |
| 1 | 1 | 2 | 5.9313 | 185. | 4 | 2 | -2 | 2.8889 | 32. | 5 | 0 | -6 | 2.1702 | 22. |
| 0 | 0 | 3 | 5.8454 | 18. | 5 | 1 | 1 | 2.8681 | 18. | 2 | 2 | -7 | 2.1663 | 17. |
| 2 | 1 | -1 | 5.7777 | 187. | 2 | 3 | -2 | 2.8556 | 17. | 1 | 0 | 8 | 2.1555 | 8. |
| 2 | 1 | 1 | 5.6436 | 129. | 5 | 0 | 2 | 2.8551 | 9. | 3 | 4 | -2 | 2.1501 | 17. |
| 2 | 0 | 2 | 5.6417 | 170. | 0 | 2 | 5 | 2.8500 | 11. | 5 | 3 | 2 | 2.1479 | 19. |
| 1 | 0 | -3 | 5.5532 | 81. | 0 | 3 | 3 | 2.8472 | 12. | 4 | 2 | -6 | 2.1412 | 36. |
| 1 | 0 | 3 | 5.3769 | 54. | 3 | 1 | -5 | 2.8338 | 13. | 7 | 1 | 0 | 2.1401 | 22. |
| 2 | 1 | -2 | 5.0643 | 122. | 4 | 1 | -4 | 2.8330 | 9. | 0 | 1 | 8 | 2.1390 | 19. |
| 0 | 1 | 3 | 5.0175 | 9. | 2 | 3 | 2 | 2.8226 | 72. | 4 | 3 | 4 | 2.1331 | 21. |
| 3 | 0 | -1 | 4.9781 | 30. | 1 | 2 | -5 | 2.8222 | 15. | 1 | 1 | -8 | 2.1323 | 18. |
| 0 | 2 | 0 | 4.8900 | 62. | 4 | 2 | 2 | 2.8218 | 24. | 2 | 2 | 7 | 2.1168 | 10. |
| 2 | 1 | 2 | 4.8869 | 111. | 5 | 1 | -2 | 2.8181 | 14. | 1 | 4 | 4 | 2.1082 | 24. |
| 3 | 0 | 1 | 4.8499 | 57. | 3 | 2 | -4 | 2.7983 | 32. | 7 | 0 | 2 | 2.1038 | 13. |
| 1 | 1 | -3 | 4.8290 | 206. | 1 | 3 | 3 | 2.7876 | 10. | 7 | 1 | -2 | 2.1021 | 14. |
| 2 | 0 | -3 | 4.7633 | 17. | 1 | 1 | -6 | 2.7778 | 19. | 2 | 3 | 6 | 2.0741 | 10. |
| 1 | 1 | 3 | 4.7117 | 191. | 5 | 0 | -3 | 2.7743 | 10. | 2 | 4 | -4 | 2.0699 | 15. |
| 0 | 2 | 1 | 4.7103 | 25. | 3 | 3 | 0 | 2.7495 | 9. | 6 | 1 | -5 | 2.0687 | 12. |
| 1 | 2 | 0 | 4.6594 | 278. | 5 | 1 | 2 | 2.7407 | 12. | 4 | 4 | 0 | 2.0621 | 15. |
| 2 | 0 | 3 | 4.5456 | 24. | 1 | 1 | 6 | 2.7326 | 41. | 6 | 2 | -4 | 2.0501 | 17. |
| 3 | 1 | 0 | 4.5344 | 196. | 3 | 3 | -1 | 2.7272 | 20. | 5 | 2 | 5 | 2.0490 | 14. |
| 1 | 2 | -1 | 4.5197 | 25. | 4 | 2 | -3 | 2.7253 | 9. | 2 | 4 | 4 | 2.0448 | 12. |
| 3 | 0 | -2 | 4.5163 | 186. | 3 | 1 | 5 | 2.7183 | 15. | 5 | 1 | 6 | 2.0228 | 15. |
| 1 | 2 | 1 | 4.4868 | 30. | 4 | 1 | 4 | 2.7104 | 10. | 0 | 4 | 5 | 2.0057 | 11. |
| 3 | 1 | -1 | 4.4364 | 102. | 3 | 2 | 4 | 2.7081 | 16. | 7 | 2 | 0 | 2.0012 | 17. |
| 3 | 1 | 1 | 4.3450 | 70. | 2 | 2 | -5 | 2.7068 | 8. | 4 | 3 | 5 | 1.9981 | 13. |
| 3 | 0 | 2 | 4.3294 | 260. | 3 | 3 | 1 | 2.7056 | 13. | 1 | 4 | -5 | 1.9960 | 24. |
| 2 | 1 | -3 | 4.2824 | 111. | 2 | 1 | -6 | 2.6710 | 23. | 4 | 4 | 2 | 1.9958 | 15. |
| 1 | 0 | -4 | 4.2706 | 216. | 3 | 3 | -2 | 2.6433 | 69. | 5 | 0 | -7 | 1.9889 | 25. |
| 1 | 0 | 4 | 4.1626 | 14. | 4 | 2 | 3 | 2.6418 | 12. | 0 | 3 | 7 | 1.9864 | 10. |
| 1 | 2 | -2 | 4.1398 | 148. | 0 | 3 | 4 | 2.6160 | 25. | 6 | 2 | 4 | 1.9794 | 18. |
| 2 | 2 | 0 | 4.1243 | 116. | 3 | 3 | 2 | 2.6043 | 21. | 6 | 1 | 5 | 1.9792 | 8. |
| 3 | 1 | -2 | 4.1002 | 30. | 5 | 2 | 0 | 2.6004 | 12. | 6 | 3 | -2 | 1.9783 | 11. |
| 1 | 2 | 2 | 4.0896 | 224. | 2 | 1 | 6 | 2.5923 | 20. | 1 | 2 | 8 | 1.9723 | 9. |
| 0 | 1 | 4 | 4.0005 | 84. | 1 | 3 | -4 | 2.5913 | 43. | 4 | 2 | -7 | 1.9651 | 8. |
| 2 | 2 | 1 | 3.9916 | 229. | 5 | 0 | -4 | 2.5744 | 16. | 5 | 3 | 4 | 1.9635 | 16. |
| 3 | 1 | 2 | 3.9588 | 895. | 5 | 1 | 3 | 2.5718 | 39. | 4 | 4 | -3 | 1.9608 | 16. |
| 3 | 0 | -3 | 3.9465 | 35. | 1 | 3 | 4 | 2.5666 | 23. | 7 | 1 | -4 | 1.9601 | 14. |
| 1 | 1 | -4 | 3.9138 | 327. | 3 | 2 | -5 | 2.5327 | 49. | 3 | 4 | 4 | 1.9544 | 52. |
| 2 | 0 | -4 | 3.8891 | 16. | 4 | 2 | -4 | 2.5321 | 28. | 4 | 0 | -8 | 1.9445 | 9. |
| 4 | 0 | 0 | 3.8382 | 44. | 5 | 2 | -2 | 2.5215 | 16. | 6 | 2 | -5 | 1.9424 | 9. |
| 1 | 1 | 4 | 3.8301 | 110. | 2 | 3 | -4 | 2.4984 | 8. | 4 | 4 | 3 | 1.9290 | 13. |
| 4 | 0 | -1 | 3.7880 | 286. | 1 | 0 | -7 | 2.4918 | 19. | 2 | 4 | 5 | 1.9275 | 8. |
| 2 | 2 | -2 | 3.7701 | 195. | 6 | 0 | -2 | 2.4890 | 8. | 4 | 3 | -6 | 1.9231 | 23. |
| 0 | 2 | 3 | 3.7507 | 17. | 6 | 1 | 0 | 2.4755 | 11. | 8 | 0 | 0 | 1.9191 | 9. |
| 2 | 2 | 2 | 3.6951 | 65. | 6 | 1 | -1 | 2.4673 | 11. | 5 | 4 | 0 | 1.9127 | 10. |
| 1 | 2 | -3 | 3.6699 | 30. | 5 | 2 | 2 | 2.4656 | 39. | 0 | 1 | 9 | 1.9109 | 14. |
| 3 | 1 | -3 | 3.6598 | 118. | 3 | 3 | 3 | 2.4634 | 9. | 0 | 5 | 2 | 1.9091 | 11. |
| 1 | 2 | 3 | 3.6177 | 151. | 2 | 3 | 4 | 2.4546 | 10. | 1 | 5 | -2 | 1.8969 | 18. |
| 2 | 1 | -4 | 3.6138 | 98. | 1 | 0 | 7 | 2.4536 | 11. | 3 | 2 | -8 | 1.8916 | 11. |
| 4 | 0 | -2 | 3.5805 | 1000. | 4 | 1 | 5 | 2.4483 | 8. | 8 | 1 | 0 | 1.8832 | 11. |
| 4 | 1 | 0 | 3.5729 | 172. | 4 | 2 | 4 | 2.4434 | 9. | 5 | 4 | -2 | 1.8806 | 12. |
| 3 | 2 | 0 | 3.5355 | 249. | 6 | 1 | 1 | 2.4354 | 16. | 7 | 1 | -5 | 1.8666 | 13. |
| 4 | 1 | -1 | 3.5323 | 172. | 6 | 0 | 2 | 2.4249 | 14. | 5 | 1 | 7 | 1.8619 | 10. |
| 3 | 1 | 3 | 3.5105 | 22. | 0 | 4 | 1 | 2.4216 | 16. | 8 | 1 | -2 | 1.8595 | 15 |
| 0 | 0 | 5 | 3.5073 | 27. | 1 | 4 | 0 | 2.4146 | 13. | 8 | 0 | 2 | 1.8560 | 11. |
| 3 | 2 | -1 | 3.4885 | 12. | 2 | 2 | -6 | 2.4145 | 11. | 5 | 3 | 5 | 1.8555 | 9. |
| 2 | 1 | 4 | 3.4850 | 26. | 6 | 1 | -2 | 2.4121 | 9. | 0 | 5 | 3 | 1.8549 | 17. |
| 4 | 1 | 1 | 3.4705 | 43. | 4 | 3 | -2 | 2.4105 | 12. | 8 | 0 | -3 | 1.8501 | 14. |
| 1 | 0 | -5 | 3.4558 | 10. | 3 | 1 | 6 | 2.4099 | 28. | 2 | 5 | 2 | 1.8481 | 9. |
| 4 | 0 | 2 | 3.4551 | 193. | 1 | 4 | -1 | 2.3945 | 34. | 6 | 1 | 6 | 1.8467 | 20. |
| 3 | 2 | 1 | 3.4435 | 65. | 1 | 4 | 1 | 2.3895 | 8. | 4 | 1 | 8 | 1.8319 | 15. |
| 3 | 0 | -4 | 3.4122 | 57. | 0 | 3 | 5 | 2.3878 | 24. | 8 | 1 | 2 | 1.8235 | 10. |
| 2 | 2 | -3 | 3.4121 | 12. | 5 | 1 | 4 | 2.3853 | 26. | 0 | 3 | 8 | 1.8191 | 15. |
| 4 | 1 | -2 | 3.3622 | 134. | 4 | 0 | -6 | 2.3816 | 12. | 8 | 1 | -3 | 1.8179 | 13. |
| 2 | 2 | 3 | 3.3293 | 66. | 2 | 2 | 6 | 2.3559 | 20. | 7 | 3 | -1 | 1.8176 | 16. |
| 3 | 2 | -2 | 3.3177 | 58. | 2 | 0 | 7 | 2.3482 | 36. | 3 | 5 | -2 | 1.7949 | 12. |
| 0 | 1 | 5 | 3.3014 | 36. | 1 | 3 | 5 | 2.3477 | 17. | 4 | 3 | -7 | 1.7925 | 10. |
| 0 | 2 | 4 | 3.2643 | 191. | 2 | 4 | 0 | 2.3297 | 23. | 8 | 1 | 3 | 1.7681 | 11. |
| 1 | 1 | -5 | 3.2583 | 13. | 1 | 4 | 2 | 2.3234 | 29. | 6 | 4 | 0 | 1.7677 | 9. |
| 4 | 1 | 2 | 3.2578 | 97. | 6 | 1 | -3 | 2.3189 | 24. | 8 | 1 | -4 | 1.7610 | 15. |
| 3 | 0 | 4 | 3.2524 | 9. | 2 | 4 | -1 | 2.3138 | 8. | 4 | 5 | 0 | 1.7427 | 12. |
| 3 | 2 | 2 | 3.2415 | 30. | 6 | 0 | 3 | 2.3034 | 13. | 2 | 5 | 4 | 1.7323 | 11. |
| 3 | 1 | -4 | 3.2217 | 9. | 3 | 3 | 4 | 2.3025 | 12. | 6 | 3 | 5 | 1.7177 | 10. |
| 0 | 3 | 1 | 3.2051 | 26. | 5 | 1 | -5 | 2.3014 | 23. | 4 | 5 | 2 | 1.7022 | 9. |
| 1 | 1 | 5 | 3.1978 | 53. | 3 | 0 | -7 | 2.2944 | 32. | 6 | 4 | 3 | 1.6766 | 11. |
| 1 | 3 | 0 | 3.1889 | 67. | 3 | 2 | -6 | 2.2907 | 13. | 2 | 5 | -5 | 1.6759 | 13. |
| 1 | 2 | 4 | 3.1697 | 72. | 4 | 0 | 6 | 2.2728 | 18. | 1 | 3 | -9 | 1.6702 | 12. |
| 1 | 3 | -1 | 3.1430 | 27. | 6 | 2 | 0 | 2.2672 | 12. | 9 | 1 | 1 | 1.6653 | 11. |
| 4 | 0 | 3 | 3.1393 | 21. | 6 | 2 | -1 | 2.2609 | 8. | 3 | 4 | 7 | 1.6388 | 9. |
| 2 | 0 | 5 | 3.1332 | 79. | 2 | 4 | -2 | 2.2598 | 18. | 9 | 0 | -4 | 1.6165 | 12. |
| 1 | 3 | 1 | 3.1319 | 44. | 2 | 3 | 5 | 2.2590 | 18. | 9 | 2 | 0 | 1.6107 | 8. |
| 4 | 1 | -3 | 3.1117 | 31. | 6 | 0 | -4 | 2.2581 | 12. | 2 | 5 | -6 | 1.5991 | 9. |
| 3 | 1 | 4 | 3.0862 | 18. | 0 | 4 | 3 | 2.2556 | 8. | 9 | 1 | 4 | 1.5447 | 9. |
| 2 | 1 | -5 | 3.0844 | 58. | 2 | 4 | 2 | 2.2434 | 9. | 5 | 4 | -7 | 1.5429 | 11. |
| 3 | 2 | -3 | 3.0711 | 75. | 1 | 4 | -3 | 2.2377 | 9. | | | | | |

Drying of the product at a temperature of ≤353°K led to polymorph I, whereas at a temperature of >353°K polymorph II was also obtained, its proportion being dependent on the drying time.

### Example 3: Polimorph II of arasertaconazole mononitrate (III)

Polymorph II was obtained by heating the solvated form with ½ mole of acetone (IV) at 354.2°K. This value corresponds to the start temperature of Differential Thermal Analysis (DTA) and Thermogravimetry (TG). Polymorph II was also obtained by heating polymorph I at 373°K over a period from 2 to 24h. DTA, TG or X-Ray Powder Diffraction (XRD) analysis showed this conversion. Similarly, polymorph II as a pure form was obtained by crystallization from polymorph I or from **(IV)** in water (Tables 5 and 6).

**Table 5. Crystal data and structure refinements for polymorph II of arasertaconazole mononitrate (III)**

| | |
|---|---|
| Molecular formula | C₂₀H₁₆Cl₃N₃O₄S |
| Molecular weight | 500.77 |
| Wavelength (Å) | 0.71069 |
| Crystal system, space group | Orthorhombic, P2₁2₁2₁ |

| Unit cell dimensions | |
|---|---|
| A (Å) | 9.3550(10) |
| B (Å) | 9.5010(10) |
| C (Å) | 25.3810(10) |
| Volume (Å³) | 2255.9(3) |
| Z, Density (calculated) | 4, 1.474 mg/m³ |
| µ (mm⁻¹) | 0.531 |
| F(000) | 1024 |
| Crystal size (mm) | 0.1 x 0.2 x 0.2 |
| θ Range | 1.60 - 25.00 degrees |
| Index ranges | 0≤h≤11, 0≤k≤10, 0≤I≤30 |
| Reflections collected / Independent reflections | 5610 / 1976 [R(int) = 0.10] |
| Integrity at 2θ | 85.5% |
| Data / parameters | 1976/283 |
| Goodness-of-Fit F² | 0.912 |
| Final R indices [1>2sigma(I)] | R1 = 0.048, wR2 = 0.104 |
| R indices (all data) | R1 = 0.141, wR2 = 0.136 |
| Absolute structure parameter | -0.11(16) |
| Largest difference peak and hole | 0.241 and -0.136 e.A⁻³ |

### Differential Scanning Calorimetry (DSC)

Only an endothermic peak was recorded at 171.5°C, i.e., the characteristic melting peak within the range of 170°-172°C, Figure 2.

**Table 6. X-ray diffraction peak intensities and spacing for polymorph II of arasertaconazole mononitrate (III)**

| **h** | **k** | **l** | **d** | **l** | **H** | **k** | **I** | **d** | **l** | **h** | **k** | **l** | **d** | **l** |
|---|---|---|---|---|---|---|---|---|---|---|---|---|---|---|
| 0 | 1 | 1 | 8.8980 | 74. | 0 | 1 | 8 | 3.0093 | 27. | 1 | 1 | 11 | 2.1804 | 22. |
| 1 | 0 | 1 | 8.7777 | 40. | 1 | 3 | 0 | 2.9998 | 30. | 1 | 3 | 8 | 2.1797 | 20. |
| 0 | 1 | 2 | 7.6057 | 280. | 2 | 1 | 6 | 2.9792 | 45. | 1 | 2 | 10 | 2.1772 | 11. |
| 1 | 1 | 0 | 6.6660 | 14. | 1 | 3 | 1 | 2.9790 | 9. | 2 | 2 | 9 | 2.1529 | 10. |
| 1 | 1 | 1 | 6.4473 | 183. | 0 | 3 | 3 | 2.9660 | 26. | 0 | 4 | 5 | 2.1514 | 15. |
| 0 | 0 | 4 | 6.3453 | 85. | 2 | 2 | 4 | 2.9507 | 31. | 3 | 3 | 3 | 2.1491 | 9. |
| 0 | 1 | 3 | 6.3184 | 40. | 3 | 1 | 1 | 2.9428 | 141. | 3 | 2 | 7 | 2.1166 | 24. |
| 1 | 0 | 3 | 6.2749 | 10. | 1 | 3 | 2 | 2.9193 | 106. | 0 | 3 | 9 | 2.1061 | 18. |
| 1 | 1 | 2 | 5.9014 | 58. | 3 | 1 | 2 | 2.8852 | 41. | 1 | 4 | 5 | 2.0966 | 18. |
| 0 | 1 | 4 | 5.2767 | 100. | 0 | 2 | 7 | 2.8822 | 10. | 4 | 2 | 1 | 2.0911 | 26. |
| 1 | 0 | 4 | 5.2513 | 28. | 1 | 1 | 8 | 2.8647 | 10. | 4 | 2 | 2 | 2.0701 | 42. |
| 1 | 1 | 3 | 5.2360 | 636. | 0 | 3 | 4 | 2.8337 | 18. | 1 | 0 | 12 | 2.0630 | 8. |
| 0 | 2 | 0 | 4.7505 | 41. | 2 | 2 | 5 | 2.7861 | 23. | 1 | 3 | 9 | 2.0547 | 13. |
| 0 | 2 | 1 | 4.6694 | 16. | 2 | 1 | 7 | 2.7436 | 8. | 3 | 1 | 9 | 2.0427 | 32. |
| 2 | 0 | 1 | 4.6000 | 66. | 1 | 3 | 4 | 2.7120 | 37. | 4 | 2 | 3 | 2.0366 | 11. |
| 1 | 1 | 4 | 4.5960 | 225. | 0 | 3 | 5 | 2.6869 | 10. | 3 | 3 | 5 | 2.0355 | 20. |
| 0 | 1 | 5 | 4.4772 | 83. | 2 | 2 | 6 | 2.6180 | 10. | 1 | 2 | 11 | 2.0262 | 18. |
| 1 | 0 | 5 | 4.4617 | 160. | 2 | 3 | 1 | 2.6086 | 19. | 2 | 1 | 11 | 2.0219 | 36. |
| 0 | 2 | 2 | 4.4490 | 382. | 3 | 2 | 0 | 2.6069 | 10. | 1 | 1 | 12 | 2.0160 | 10. |
| 1 | 2 | 0 | 4.2357 | 21. | 1 | 1 | 9 | 2.5972 | 14. | 3 | 2 | 8 | 2.0142 | 27. |
| 0 | 0 | 6 | 4.2302 | 89. | 3 | 1 | 5 | 2.5589 | 11. | 2 | 4 | 4 | 2.0089 | 20. |
| 0 | 2 | 3 | 4.1422 | 312. | 3 | 2 | 2 | 2.5536 | 23. | 4 | 1 | 6 | 2.0009 | 10. |
| 2 | 1 | 1 | 4.1403 | 21. | 0 | 0 | 10 | 2.5381 | 9. | 0 | 3 | 10 | 1.9806 | 9. |
| 2 | 0 | 3 | 4.0935 | 18. | 0 | 3 | 6 | 2.5352 | 9. | 2 | 4 | 5 | 1.9545 | 8. |
| 1 | 1 | 5 | 4.0385 | 175. | 2 | 1 | 8 | 2.5308 | 9. | 3 | 1 | 10 | 1.9275 | 10. |
| 2 | 1 | 2 | 3.9843 | 44. | 2 | 3 | 3 | 2.5049 | 9. | 2 | 2 | 11 | 1.8971 | 17. |
| 0 | 1 | 6 | 3.8644 | 23. | 1 | 3 | 6 | 2.4470 | 10. | 4 | 3 | 1 | 1.8762 | 15. |
| 1 | 0 | 6 | 3.8544 | 171. | 2 | 3 | 4 | 2.4236 | 8. | 1 | 1 | 13 | 1.8737 | 12. |
| 0 | 2 | 4 | 3.8028 | 107. | 0 | 4 | 1 | 2.3649 | 12. | 3 | 4 | 2 | 1.8689 | 13. |
| 1 | 2 | 3 | 3.7875 | 45. | 3 | 0 | 7 | 2.3642 | 9. | 0 | 3 | 11 | 1.8649 | 9. |
| 2 | 0 | 4 | 3.7651 | 315. | 1 | 2 | 9 | 2.3474 | 12. | 1 | 5 | 2 | 1.8424 | 17. |
| 2 | 1 | 3 | 3.7594 | 176. | 2 | 3 | 5 | 2.3299 | 26. | 2 | 4 | 7 | 1.8287 | 10. |
| 1 | 1 | 6 | 3.5717 | 47. | 4 | 0 | 1 | 2.3289 | 10. | 5 | 1 | 2 | 1.8168 | 9. |
| 1 | 2 | 4 | 3.5229 | 31. | 3 | 2 | 5 | 2.3190 | 8. | 0 | 0 | 14 | 1.8129 | 31. |
| 2 | 1 | 4 | 3.5002 | 128. | 1 | 3 | 7 | 2.3113 | 10. | 5 | 1 | 3 | 1.7940 | 10. |
| 2 | 0 | 5 | 3.4398 | 34. | 3 | 1 | 7 | 2.2943 | 33. | 2 | 2 | 12 | 1.7858 | 9. |
| 0 | 1 | 7 | 3.3876 | 1000. | 1 | 4 | 1 | 2.2928 | 10. | 1 | 4 | 9 | 1.7834 | 16. |
| 1 | 0 | 7 | 3.3808 | 25. | 4 | 1 | 1 | 2.2619 | 12. | 4 | 3 | 5 | 1.7641 | 14. |
| 2 | 2 | 1 | 3.3046 | 159. | 0 | 3 | 8 | 2.2414 | 14. | 4 | 2 | 8 | 1.7501 | 9. |
| 1 | 2 | 5 | 3.2522 | 54. | 0 | 2 | 10 | 2.2386 | 9. | 1 | 1 | 14 | 1.7494 | 17. |
| 2 | 1 | 5 | 3.2344 | 10. | 2 | 0 | 10 | 2.2308 | 14. | 1 | 3 | 12 | 1.7286 | 20. |
| 2 | 2 | 2 | 3.2237 | 54. | 2 | 3 | 6 | 2.2289 | 10. | 2 | 5 | 3 | 1.7236 | 9. |
| 1 | 1 | 7 | 3.1852 | 32. | 3 | 3 | 0 | 2.2220 | 26. | 2 | 2 | 13 | 1.6846 | 10. |
| 0 | 0 | 8 | 3.1726 | 162. | 3 | 2 | 6 | 2.2193 | 36. | 5 | 1 | 6 | 1.6840 | 13 |
| 0 | 2 | 6 | 3.1592 | 77. | 3 | 3 | 1 | 2.2135 | 10. | 0 | 1 | 15 | 1.6659 | 12. |
| 2 | 2 | 3 | 3.1010 | 119. | 4 | 0 | 4 | 2.1944 | 29. | 3 | 5 | 2 | 1.6096 | 8. |
| 0 | 3 | 2 | 3.0728 | 39. | 3 | 3 | 2 | 2.1887 | 11. | 1 | 6 | 1 | 1.5583 | 9. |

### Stability

Polymorph II was stable against humidity and at a temperature below 423°K. Polymorph II remained stable in the course of a 1-month test at ambient temperature or humidity and after 1 day at 25°C/80RH. No transition from II to I was observed during this 1-month test. The start of endothermic peak temperature occurred at 437.6°K, but the first recorded weight loss began at 449°K from DTA analysis. Polymorph II placed in a furnace at 423°K started decomposition after 2 hours.

### Example 4: Determination of apparent density and compacted density

The determination of apparent and compacted densities was carried out with a Stampf STAV 2003 volumeter equipped with a 100-mL glass volumetric flask.

### Procedure

(i) The flask was filled with a volume between 50 and 100mL of test sample.
(ii) The volume occupied before the test (mL) was recorded.
(iii) 2500 strokes were performed with the volumeter.
(iv) The volume occupied after the test (mL) was recorded.
(v) The amount of analyzed sample (g) was weighed.

### Calculation

(i) Apparent density (g/mL) = weight / initial volume
(ii) Compacted density (g/mL) = weight / final volume

### Results

**Table 7. Apparent density and compacted density of polymorph I and polymorph II of arasertaconazole mononitrate (III)**

| | Polymorph I | Polymorph II |
|---|---|---|
| Apparent density (g/mL) | 0.78 | 0.39 |
| Compacted density (g/mL) | 1.08 | 0.53 |

### Example 5: 1 % Cream Formulation (100g)

| Polymorph II of arasertaconazole mononitrate **(III)** | 1.00g |
|---|---|
| Palmitic and stearic acid mono- and diglyceride | 6.00g |
| Cetostearyl alcohol with 20 mol of ethylene oxide | 1.00g |
| Oleic acid decyl ester | 5.00g |
| Undecylenic acid monoethanolamine | 2.00g |
| Carbomer | 1.00g |
| Triethanolamine | 0.60g |
| Methylparaben | 0.15g |
| Propylparaben | 0.05g |
| Distilled water q.s. to | 100.00g |

### Example 6: 1 % Gel Formulation (100g)

| Polymorph II of arasertaconazole mononitrate (III) | 1.00g |
|---|---|
| Propylen glycol | 10.00g |
| Carbomer | 1.00g |
| Tween 20 | 0.10g |
| Phenoxyethanol | 0.35g |
| EDTA disodium | 0.15g |
| Citric acid | 0.25g |
| 1 N Sodium hydroxide | 1.50g |
| Triethanolamine | 1.55g |
| Distilled water q.s. to | 100.00g |

## Claims

1. Polymorph II of an antifungal compound of formula **(III):** 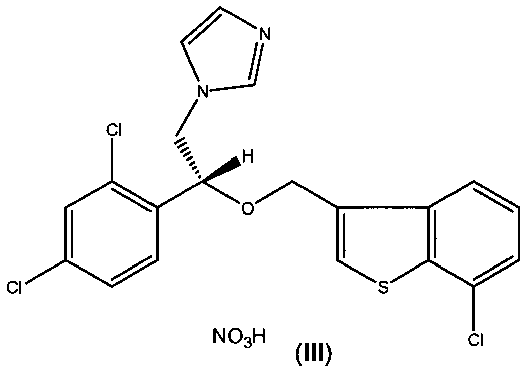 **characterized by** presenting:
(i) a melting point range of 170°-172°C;
(ii) the following crystallographic characteristics:
| | |
|---|---|
| Wavelength (Å) | 0.71069 |
| Crystal system, space group | Orthorhombic, P2₁2₁2₁ |
| Unit cell dimensions | |
|---|---|
| A (Å) | 9.3550(10) |
| B (Å) | 9.5010(10) |
| C (Å) | 25.3810(10) |
| Volume (Å³) | 2255.9(3) |
| Z, Density (calculated) | 4, 1.474 mg/m³ |
| µ (mm⁻¹) | 0.531 |
| F(000) | 1024 |
| Crystal size (mm) | 0.1 x 0.2 x 0.2 |
| θ Range | 1.60 - 25.00 degrees |
| Index range | 0≤h≤11, 0≤k≤10, 0≤l≤30 |
| Reflections collected / Independent reflections | 5610 / 1976 [R(int) = 0.10] |
| Integrity at 2θ | 85.5% |
| Data / parameters | 1976/283 |
| Goodness-of-Fit F² | 0.912 |
| Final R indices [l>2sigma(l)] | R1 = 0.048, wR2 = 0.104 |
| R indices (all data) | R1 = 0.141, wR2 = 0.136 |
| Absolute structure parameter | -0.11(16) |
| Largest difference peak and hole | 0.241 and -0.136 e.A⁻³ |
and
(iii) the following X-ray diffraction peak intensities and spacing:
| **h** | **k** | **l** | **D** | **l** | **h** | **k** | **l** | **d** | **l** | **h** | **k** | **l** | **d** | **l** |
|---|---|---|---|---|---|---|---|---|---|---|---|---|---|---|
| 0 | 1 | 1 | 8.8980 | 74. | 0 | 1 | 8 | 3.0093 | 27. | 1 | 1 | 11 | 2.1804 | 22. |
| 1 | 0 | 1 | 8.7777 | 40. | 1 | 3 | 0 | 2.9998 | 30. | 1. | 3 | 8 | 2.1797 | 20. |
| 0 | 1 | 2 | 7.6057 | 280. | 2 | 1 | 6 | 2.9792 | 45. | 1 | 2 | 10 | 2.1772 | 11. |
| 1 | 1 | 0 | 6.6660 | 14. | 1 | 3 | 1 | 2.9790 | 9. | 2 | 2 | 9 | 2.1529 | 10. |
| 1 | 1 | 1 | 6.4473 | 183. | 0 | 3 | 3 | 2.9660 | 26. | 0 | 4 | 5 | 2.1514 | 15. |
| 0 | 0 | 4 | 6.3453 | 85. | 2 | 2 | 4 | 2.9507 | 31. | 3 | 3 | 3 | 2.1491 | 9. |
| 0 | 1 | 3 | 6.3184 | 40. | 3 | 1 | 1 | 2.9428 | 141. | 3 | 2 | 7 | 2.1166 | 24. |
| 1 | 0 | 3 | 6.2749 | 10. | 1 | 3 | 2 | 2.9193 | 106. | 0 | 3 | 9 | 2.1061 | 18. |
| 1 | 1 | 2 | 5.9014 | 58. | 3 | 1 | 2 | 2.8852 | 41. | 1 | 4 | 5 | 2.0966 | 18. |
| 0 | 1 | 4 | 5.2767 | 100. | 0 | 2 | 7 | 2.8822 | 10. | 4 | 2 | 1 | 2.0911 | 26. |
| 1 | 0 | 4 | 5.2513 | 28. | 1 | 1 | 8 | 2.8647 | 10. | 4 | 2 | 2 | 2.0701 | 42. |
| 1 | 1 | 3 | 5.2360 | 636. | 0 | 3 | 4 | 2.8337 | 18. | 1 | 0 | 12 | 2.0630 | 8. |
| 0 | 2 | 0 | 4.7505 | 41. | 2 | 2 | 5 | 2.7861 | 23. | 1 | 3 | 9 | 2.0547 | 13. |
| 0 | 2 | 1 | 4.6694 | 16. | 2 | 1 | 7 | 2.7436 | 8. | 3 | 1 | 9 | 2.0427 | 32. |
| 2 | 0 | 1 | 4.6000 | 66. | 1 | 3 | 4 | 2.7120 | 37. | 4 | 2 | 3 | 2.0366 | 11. |
| 1 | 1 | 4 | 4.5960 | 225. | 0 | 3 | 5 | 2.6869 | 10. | 3 | 3 | 5 | 2.0355 | 20. |
| 0 | 1 | 5 | 4.4772 | 83. | 2 | 2 | 6 | 2.6180 | 10. | 1 | 2 | 11 | 2.0262 | 18. |
| 1 | 0 | 5 | 4.4617 | 160. | 2 | 3 | 1 | 2.6086 | 19. | 2 | 1 | 11 | 2.0219 | 36. |
| 0 | 2 | 2 | 4.4490 | 382. | 3 | 2 | 0 | 2.6069 | 10. | 1 | 1 | 12 | 2.0160 | 10. |
| 1 | 2 | 0 | 4.2357 | 21. | 1 | 1 | 9 | 2.5972 | 14. | 3 | 2 | 8 | 2.0142 | 27. |
| 0 | 0 | 6 | 4.2302 | 89. | 3 | 1 | 5 | 2.5589 | 11. | 2 | 4 | 4 | 2.0089 | 20. |
| 0 | 2 | 3 | 4.1422 | 312. | 3 | 2 | 2 | 2.5536 | 23. | 4 | 1 | 6 | 2.0009 | 10. |
| 2 | 1 | 1 | 4.1403 | 21. | 0 | 0 | 10 | 2.5381 | 9. | 0 | 3 | 10 | 1.9806 | 9. |
| 2 | 0 | 3 | 4.0935 | 18. | 0 | 3 | 6 | 2.5352 | 9. | 2 | 4 | 5 | 1.9545 | 8. |
| 1 | 1 | 5 | 4.0385 | 175. | 2 | 1 | 8 | 2.5308 | 9. | 3 | 1 | 10 | 1.9275 | 10. |
| 2 | 1 | 2 | 3.9843 | 44. | 2 | 3 | 3 | 2.5049 | 9. | 2 | 2 | 11 | 1.8971 | 17. |
| 0 | 1 | 6 | 3.8644 | 23. | 1 | 3 | 6 | 2.4470 | 10. | 4 | 3 | 1 | 1.8762 | 15. |
| 1 | 0 | 6 | 3.8544 | 171. | 2 | 3 | 4 | 2.4236 | 8. | 1 | 1 | 13 | 1.8737 | 12. |
| 0 | 2 | 4 | 3.8028 | 107. | 0 | 4 | 1 | 2.3649 | 12. | 3 | 4 | 2 | 1.8689 | 13. |
| 1 | 2 | 3 | 3.7875 | 45. | 3 | 0 | 7 | 2.3642 | 9. | 0 | 3 | 11 | 1.8649 | 9. |
| 2 | 0 | 4 | 3.7651 | 315. | 1 | 2 | 9 | 2.3474 | 12. | 1 | 5 | 2 | 1.8424 | 17. |
| 2 | 1 | 3 | 3.7594 | 176. | 2 | 3 | 5 | 2.3299 | 26. | 2 | 4 | 7 | 1.8287 | 10. |
| 1 | 1 | 6 | 3.5717 | 47. | 4 | 0 | 1 | 2.3289 | 10. | 5 | 1 | 2 | 1.8168 | 9. |
| 1 | 2 | 4 | 3.5229 | 31. | 3 | 2 | 5 | 2.3190 | 8. | 0 | 0 | 14 | 1.8129 | 31. |
| 2 | 1 | 4 | 3.5002 | 128. | 1 | 3 | 7 | 2.3113 | 10. | 5 | 1 | 3 | 1.7940 | 10. |
| 2 | 0 | 5 | 3.4398 | 34. | 3 | 1 | 7 | 2.2943 | 33. | 2 | 2 | 12 | 1.7858 | 9. |
| 0 | 1 | 7 | 3.3876 | 1000. | 1 | 4 | 1 | 2.2928 | 10. | 1 | 4 | 9 | 1.7834 | 16. |
| 1 | 0 | 7 | 3.3808 | 25. | 4 | 1 | 1 | 2.2619 | 12. | 4 | 3 | 5 | 1.7641 | 14. |
| 2 | 2 | 1 | 3.3046 | 159. | 0 | 3 | 8 | 2.2414 | 14. | 4 | 2 | 8 | 1.7501 | 9. |
| 1 | 2 | 5 | 3.2522 | 54. | 0 | 2 | 10 | 2.2386 | 9. | 1 | 1 | 14 | 1.7494 | 17. |
| 2 | 1 | 5 | 3.2344 | 10. | 2 | 0 | 10 | 2.2308 | 14. | 1 | 3 | 12 | 1.7286 | 20. |
| 2 | 2 | 2 | 3.2237 | 54. | 2 | 3 | 6 | 2.2289 | 10. | 2 | 5 | 3 | 1.7236 | 9. |
| 1 | 1 | 7 | 3.1852 | 32. | 3 | 3 | 0 | 2.2220 | 26. | 2 | 2 | 13 | 1.6846 | 10. |
| 0 | 0 | 8 | 3.1726 | 162. | 3 | 2 | 6 | 2.2193 | 36. | 5 | 1 | 6 | 1.6840 | 13 |
| 0 | 2 | 6 | 3.1592 | 77. | 3 | 3 | 1 | 2.2135 | 10. | 0 | 1 | 15 | 1.6659 | 12. |
| 2 | 2 | 3 | 3.1010 | 119. | 4 | 0 | 4 | 2.1944 | 29. | 3 | 5 | 2 | 1.6096 | 8. |
| 0 | 3 | 2 | 3.0728 | 39. | 3 | 3 | 2 | 2.1887 | 11. | 1 | 6 | 1 | 1.5583 | 9. |

2. The polymorph according to claim 1 **characterized by** a differential scanning calorimetry (DSC) thermogram showing an endothermic peak at 171.5°C.

3. A process for preparing the polymorph according to claim 1, comprising heating the solvated form of arasertaconazole mononitrate with ½ mole of acetone, of formula **(IV):** 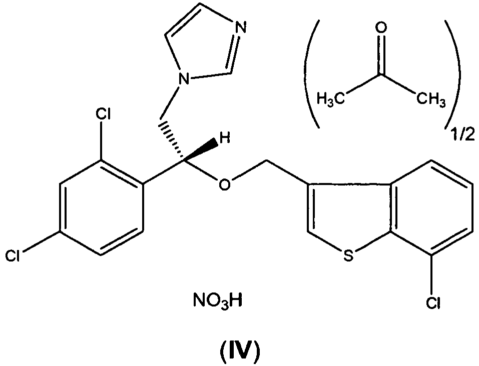 at a temperature of 354.2°K.

4. A process for preparing the polymorph according to claim 1, comprising heating polymorph I of said compound at 373°K over a period from 2 to 24h.

5. A process for preparing the polymorph according to claim 1, comprising crystallization of polymorph I or the solvated form of arasertaconazole mononitrate with ½ mole of acetone **(IV)** in water.

6. Use of the polymorph according to claim 1 for preparing a pharmaceutical composition for the treatment or prevention of skin and mucous membrane infections caused by fungi and yeasts in humans or pets.

7. A polymorph as defined in claim 1 for use in the treatment or prevention of skin and mucous membrane infections caused by fungi and yeasts.

8. Use of the polymorph according to claim 1 for the treatment or prevention of crop diseases produced by fungi and yeasts.

9. Pharmaceutical composition comprising the polymorph according to cairn 1 and pharmaceutically acceptable carriers for use in the treatment or prevention of skin and mucous membrane infections caused by fungi and yeasts in humans or pets.

10. Agricultural composition comprising the polymorph according to claim 1 and agriculturally acceptable carriers for the treatment or prevention of crop diseases produced by fungi and yeasts.

## Patentansprüche

1. Polymorph II einer antifungiellen Verbindung der Formel (III): 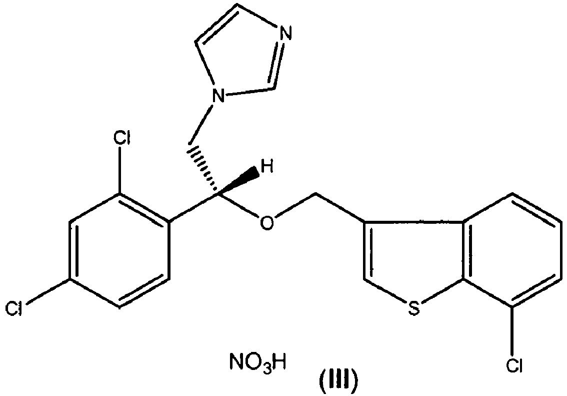 **dadurch gekennzeichnet, dass** es Folgendes aufweist:
(i) einen Schmelztemperaturbereich von 170 °C bis 172 °C;
(ii) die folgenden kristallographischen Eigenschaften :
| | |
|---|---|
| Wellenlänge (Å) | 0,71069 |
| Kristallsystem, Raumgruppe | Orthorhombisch, P2₁2₁2₁ |
| Zellabmessungen (Einheiten) | |
|---|---|
| A (Å) | 9,3550(10) |
| B(Å) | 9,5010(10) |
| C (Å) | 25,3810(10) |
| Volumen (Å³) | 2255,9(3) |
| Z, Dichte (berechnet) | 4; 1,474 mg/m³ |
| µ (mm⁻¹) | 0,531 |
| F(000) | 1024 |
| Kristallgröße (mm) | 0,1 x 0,2 x 0,2 |
| θ Bereich | 1,60 -25,00 Grad |
| Indexbereich | 0≤h≤11, 0≤k≤10, 0≤1≤30 |
| Aufgefangene Reflexionen / Unabhängige | 5610/1976 [R(int) = 0,10] |
| Reflexionen | |
| Integrität bei 2θ | 85,5% |
| Daten / Parameter | 1976/283 |
| Anpassungsgüte F² | 0,912 |
| End-R-Indizes [1 >2sigma(l)] | R1 = 0,048, wR2 = 0,104 |
| R-Indizes (Alle Daten) | R1 = 0,141, wR2=0,136 |
| Absolute Strukturparameter | -0,11(16) |
| Größte Abstände Spitze - Probe | 0,241 und -0,136 e.A⁻³ |
und
(iii) die folgenden Spitzenintensitäten und Abstände bei Röntgenbeugung:
| **h** | **k** | **l** | **D** | **l** | **h** | **k** | **l** | **d** | **l** | **h** | **k** | **l** | **d** | **l** |
|---|---|---|---|---|---|---|---|---|---|---|---|---|---|---|
| 0 | 1 | 1 | 8,8980 | 74. | 0 | 1 | 8 | 3,0093 | 27. | 1 | 1 | 11 | 2,1804 | 22. |
| 1 | 0 | 1 | 8,7777 | 40. | 1 | 3 | 0 | 2,9998 | 30. | 1 | 3 | 8 | 2,1797 | 20. |
| 0 | 1 | 2 | 7,6057 | 280. | 2 | 1 | 6 | 2,9792 | 45. | 1 | 2 | 10 | 2,1772 | 11. |
| 1 | 1 | 0 | 6,6660 | 14. | 1 | 3 | 1 | 2,9790 | 9. | 2 | 2 | 9 | 2,1529 | 10. |
| 1 | 1 | 1 | 6,4473 | 183. | 0 | 3 | 3 | 2,9660 | 26. | 0 | 4 | 5 | 2,1514 | 15. |
| 0 | 0 | 4 | 6,3453 | 85. | 2 | 2 | 4 | 2,9507 | 31. | 3 | 3 | 3 | 2,1491 | 9. |
| 0 | 1 | 3 | 6,3184 | 40. | 3 | 1 | 1 | 2,9428 | 141. | 3 | 2 | 7 | 2,1166 | 24. |
| 1 | 0 | 3 | 6,2749 | 10. | 1 | 3 | 2 | 2,9193 | 106. | 0 | 3 | 9 | 2,1061 | 18. |
| 1 | 1 | 2 | 5,9014 | 58. | 3 | 1 | 2 | 2,8852 | 41. | 1 | 4 | 5 | 2,0966 | 18. |
| 0 | 1 | 4 | 5,2767 | 100. | 0 | 2 | 7 | 2,8822 | 10. | 4 | 2 | 1 | 2,0911 | 26. |
| 1 | 0 | 4 | 5,2513 | 28. | 1 | 1 | 8 | 2,8647 | 10. | 4 | 2 | 2 | 2,0701 | 42. |
| 1 | 1 | 3 | 5,2360 | 636. | 0 | 3 | 4 | 2,8337 | 18. | 1 | 0 | 12 | 2,0630 | 8. |
| 0 | 2 | 0 | 4,7505 | 41. | 2 | 2 | 5 | 2,7861 | 23. | 1 | 3 | 9 | 2,0547 | 13. |
| 0 | 2 | 1 | 4,6694 | 16. | 2 | 1 | 7 | 2,7436 | 8. | 3 | 1 | 9 | 2,0427 | 32. |
| 2 | 0 | 1 | 4,6000 | 66. | 1 | 3 | 4 | 2,7120 | 37. | 4 | 2 | 3 | 2,0366 | 11. |
| 1 | 1 | 4 | 4,5960 | 225. | 0 | 3 | 5 | 2,6869 | 10. | 3 | 3 | 5 | 2,0355 | 20. |
| 0 | 1 | 5 | 4,4772 | 83. | 2 | 2 | 6 | 2,6180 | 10. | 1 | 2 | 11 | 2,0262 | 18. |
| 1 | 0 | 5 | 4,4617 | 160. | 2 | 3 | 1 | 2,6086 | 19. | 2 | 1 | 11 | 2,0219 | 36. |
| 0 | 2 | 2 | 4,4490 | 382. | 3 | 2 | 0 | 2,6069 | 10. | 1 | 1 | 12 | 2,0160 | 10. |
| 1 | 2 | 0 | 4,2357 | 21. | 1 | 1 | 9 | 2,5972 | 14. | 3 | 2 | 8 | 2,0142 | 27. |
| 0 | 0 | 6 | 4,2302 | 89. | 3 | 1 | 5 | 2,5589 | 11. | 2 | 4 | 4 | 2,0089 | 20. |
| 0 | 2 | 3 | 4,1422 | 312. | 3 | 2 | 2 | 2,5536 | 23. | 4 | 1 | 6 | 2,0009 | 10. |
| 2 | 1 | 1 | 4,1403 | 21. | 0 | 0 | 10 | 2,5381 | 9. | 0 | 3 | 10 | 1,9806 | 9. |
| 2 | 0 | 3 | 4,0935 | 18. | 0 | 3 | 6 | 2,5352 | 9. | 2 | 4 | 5 | 1,9545 | 8. |
| 1 | 1 | 5 | 4,0385 | 175. | 2 | 1 | 8 | 2,5308 | 9. | 3 | 1 | 10 | 1,9275 | 10. |
| 2 | 1 | 2 | 3,9843 | 44. | 2 | 3 | 3 | 2,5049 | 9. | 2 | 2 | 11 | 1,8971 | 17. |
| 0 | 1 | 6 | 3,8644 | 23. | 1 | 3 | 6 | 2,4470 | 10. | 4 | 3 | 1 | 1,8762 | 15. |
| 1 | 0 | 6 | 3,8544 | 171. | 2 | 3 | 4 | 2,4236 | 8. | 1 | 1 | 13 | 1,8737 | 12. |
| 0 | 2 | 4 | 3,8028 | 107. | 0 | 4 | 1 | 2,3649 | 12. | 3 | 4 | 2 | 1,8689 | 13. |
| 1 | 2 | 3 | 3,7875 | 45. | 3 | 0 | 7 | 2,3642 | 9. | 0 | 3 | 11 | 1,8649 | 9. |
| 2 | 0 | 4 | 3,7651 | 315. | 1 | 2 | 9 | 2,3474 | 12. | 1 | 5 | 2 | 1,8424 | 17. |
| 2 | 1 | 3 | 3,7594 | 176. | 2 | 3 | 5 | 2,3299 | 26. | 2 | 4 | 7 | 1,8287 | 10. |
| 1 | 1 | 6 | 3,5717 | 47. | 4 | 0 | 1 | 2,3289 | 10. | 5 | 1 | 2 | 1,8168 | 9. |
| 1 | 2 | 4 | 3,5229 | 31. | 3 | 2 | 5 | 2,3190 | 8. | 0 | 0 | 14 | 1,8129 | 31. |
| 2 | 1 | 4 | 3,5002 | 128. | 1 | 3 | 7 | 2,3113 | 10. | 5 | 1 | 3 | 1,7940 | 10. |
| 2 | 0 | 5 | 3,4398 | 34. | 3 | 1 | 7 | 2,2943 | 33. | 2 | 2 | 12 | 1,7858 | 9. |
| 0 | 1 | 7 | 3,3876 | 1000. | 1 | 4 | 1 | 2,2928 | 10. | 1 | 4 | 9 | 1,7834 | 16. |
| 1 | 0 | 7 | 3,3808 | 25. | 4 | 1 | 1 | 2,2619 | 12. | 4 | 3 | 5 | 1,7641 | 14. |
| 2 | 2 | 1 | 3,3046 | 159. | 0 | 3 | 8 | 2,2414 | 14. | 4 | 2 | 8 | 1,7501 | 9. |
| 1 | 2 | 5 | 3,2522 | 54. | 0 | 2 | 10 | 2,2386 | 9. | 1 | 1 | 14 | 1,7494 | 17. |
| 2 | 1 | 5 | 3,2344 | 10. | 2 | 0 | 10 | 2,2308 | 14. | 1 | 3 | 12 | 1,7286 | 20. |
| 2 | 2 | 2 | 3,2237 | 54. | 2 | 3 | 6 | 2,2289 | 10. | 2 | 5 | 3 | 1,7236 | 9. |
| 1 | 1 | 7 | 3,1852 | 32. | 3 | 3 | 0 | 2,2220 | 26. | 2 | 2 | 13 | 1,6846 | 10. |
| 0 | 0 | 8 | 3,1726 | 162. | 3 | 2 | 6 | 2,2193 | 36. | 5 | 1 | 6 | 1,6840 | 13 |
| 0 | 2 | 6 | 3,1592 | 77. | 3 | 3 | 1 | 2,2135 | 10. | 0 | 1 | 15 | 1,6659 | 12. |
| 2 | 2 | 3 | 3,1010 | 119. | 4 | 0 | 4 | 2,1944 | 29. | 3 | 5 | 2 | 1,6096 | 8. |
| 0 | 3 | 2 | 3,0728 | 39. | 3 | 3 | 2 | 2,1887 | 11. | 1 | 6 | 1 | 1,5583 | 9. |

2. Polymorph nach Anspruch 1 **dadurch gekennzeichnet, dass** es ein Thermogramm der Dynamischen Differenzkalorimetrie (DDK) aufweist, das eine endothermische Spitze bei 171,5 °C hat.

3. Verfahren zur Herstellung eines Polymorphs nach Anspruch 1, umfassend die Erhitzung der solvatisierten Form des Arasertaconazolmononitrats mit ½ Mol Aceton, der Formel (IV) 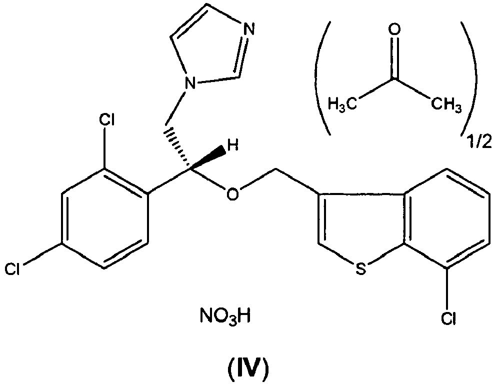 bei einer Temperatur von 354,2 °K.

4. Verfahren zur Herstellung des Polymorphs nach Anspruch 1, umfassend die Erhitzung des Polymorphs I der Verbindung bei 373 °K in einem Zeitraum von 2 bis 24 h.

5. Verfahren zur Herstellung des Polymorphs nach Anspruch 1, umfassend die Kristallisierung des Polymorphs I oder der solvatisierten Form des Arasertaconazolmononitrats mit ½ Mol Aceton (IV) in Wasser.

6. Verwendung des Polymorphs nach Anspruch 1 zur Herstellung einer pharmazeutischen Zusammensetzung für die Behandlung oder für die Prävention von Haut- bzw. Schleimhautinfektionen bei Menschen oder Haustieren, die durch Pilze und Hefen verursacht werden.

7. Polymorph nach Anspruch 1 zur Verwendung bei der Behandlung oder bei der Prävention von Haut- bzw. Schleimhautinfektionen, die durch Pilze und Hefen verursacht werden.

8. Verwendung des Polymorphs nach Anspruch 1 zur Behandlung oder zur Prävention von Pflanzenkrankheiten, die durch Pilze und Hefen verursacht werden.

9. Pharmazeutische Zusammensetzung umfassend das Polymorph nach Anspruch 1 und pharmazeutisch annehmbare Träger zur Verwendung bei der Behandlung oder bei der Prävention von Haut- bzw. Schleimhautinfektionen bei Menschen oder Haustieren, die durch Pilze und Hefen verursacht werden.

10. Landwirtschaftliche Zusammensetzung umfassend das Polymorph nach Anspruch 1 und pharmazeutisch annehmbare Träger zur Behandlung oder zur Prävention von Pflanzenkrankheiten, die durch Pilze und Hefen verursacht werden.

## Revendications

1. Polymorphe II d'une composition antifungique de formule (III): 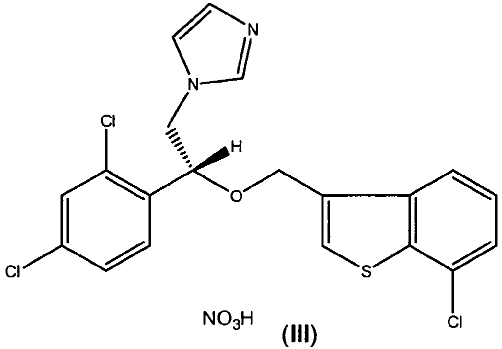 **caractérisé en ce qu'**il comprend:
(i) un intervalle de température de fusion allant de 170 °C à 172 °C;
(ii) les propriétés cristallographiques suivantes :
| | |
|---|---|
| Longueur d'onde (Å) | 0,71069 |
| Système cristallin, groupe d'espace | orthorhombique, P2₁2₁2₁ |
| Dimensions de maille unitaires | |
|---|---|
| A (A) | 9,3550(10) |
| B (Å) | 9,5010(10) |
| C (Å) | 25,3810(10) |
| Volume (Å³) | 2255,9(3) |
| Z, densité (calculée) | 4; 1,474 mg/m³ |
| µ (mm⁻¹) | 0,531 |
| F(000) | 1024 |
| Taille des cristaux (mm) | 0,1 x 0,2 x 0,2 |
| θ Intervalle | 1,60 -25,00 degrés |
| Intervalle d'indices | 0≤h≤11, 0≤k≤10, 0≤1≤30 |
| Réflexions collectées / réflexions indépendantes | 5610/1976 [R(int) = 0,10] |
| Intégrité à 2θ | 85,5% |
| Données / Paramètres | 1976/283 |
| Qualité d'ajustement F² | 0,912 |
| Indices R finals [1 >2sigma(l)] | R1 = 0,048 ; wR2 = 0,104 |
| Indices R (toutes les données) | R1 = 0,141 ; wR2=0,136 |
| Paramètre de structure absolu | -0,11(16) |
| Plus longe différence sommet-trou | 0,241 et -0,136 e.A⁻³ |
et
iii) les intensités des pics et intervalles de diffraction de rayons X suivantes:
| **h** | **k** | **l** | **D** | **l** | **h** | **k** | **l** | **d** | **l** | **h** | **k** | **l** | **d** | **l** |
|---|---|---|---|---|---|---|---|---|---|---|---|---|---|---|
| 0 | 1 | 1 | 8,8980 | 74. | 0 | 1 | 8 | 3,0093 | 27. | 1 | 1 | 11 | 2,1804 | 22. |
| 1 | 0 | 1 | 8,7777 | 40. | 1 | 3 | 0 | 2,9998 | 30. | 1 | 3 | 8 | 2,1797 | 20. |
| 0 | 1 | 2 | 7,6057 | 280. | 2 | 1 | 6 | 2,9792 | 45. | 1 | 2 | 10 | 2,1772 | 11. |
| 1 | 1 | 0 | 6,6660 | 14. | 1 | 3 | 1 | 2,9790 | 9. | 2 | 2 | 9 | 2,1529 | 10. |
| 1 | 1 | 1 | 6,4473 | 183. | 0 | 3 | 3 | 2,9660 | 26. | 0 | 4 | 5 | 2,1514 | 15. |
| 0 | 0 | 4 | 6,3453 | 85. | 2 | 2 | 4 | 2,9507 | 31. | 3 | 3 | 3 | 2,1491 | 9. |
| 0 | 1 | 3 | 6,3184 | 40. | 3 | 1 | 1 | 2,9428 | 141. | 3 | 2 | 7 | 2,1166 | 24. |
| 1 | 0 | 3 | 6,2749 | 10. | 1 | 3 | 2 | 2,9193 | 106. | 0 | 3 | 9 | 2,1061 | 18. |
| 1 | 1 | 2 | 5,9014 | 58. | 3 | 1 | 2 | 2,8852 | 41. | 1 | 4 | 5 | 2,0966 | 18. |
| 0 | 1 | 4 | 5,2767 | 100. | 0 | 2 | 7 | 2,8822 | 10. | 4 | 2 | 1 | 2,0911 | 26. |
| 1 | 0 | 4 | 5,2513 | 28. | 1 | 1 | 8 | 2,8647 | 10. | 4 | 2 | 2 | 2,0701 | 42. |
| 1 | 1 | 3 | 5,2360 | 636. | 0 | 3 | 4 | 2,8337 | 18. | 1 | 0 | 12 | 2,0630 | 8. |
| 0 | 2 | 0 | 4,7505 | 41. | 2 | 2 | 5 | 2,7861 | 23. | 1 | 3 | 9 | 2,0547 | 13. |
| 0 | 2 | 1 | 4,6694 | 16. | 2 | 1 | 7 | 2,7436 | 8. | 3 | 1 | 9 | 2,0427 | 32. |
| 2 | 0 | 1 | 4,6000 | 66. | 1 | 3 | 4 | 2,7120 | 37. | 4 | 2 | 3 | 2,0366 | 11. |
| 1 | 1 | 4 | 4,5960 | 225. | 0 | 3 | 5 | 2,6869 | 10. | 3 | 3 | 5 | 2,0355 | 20. |
| 0 | 1 | 5 | 4,4772 | 83. | 2 | 2 | 6 | 2,6180 | 10. | 1 | 2 | 11 | 2,0262 | 18. |
| 1 | 0 | 5 | 4,4617 | 160. | 2 | 3 | 1 | 2,6086 | 19. | 2 | 1 | 11 | 2,0219 | 36. |
| 0 | 2 | 2 | 4,4490 | 382. | 3 | 2 | 0 | 2,6069 | 10. | 1 | 1 | 12 | 2,0160 | 10. |
| 1 | 2 | 0 | 4,2357 | 21. | 1 | 1 | 9 | 2,5972 | 14. | 3 | 2 | 8 | 2,0142 | 27. |
| 0 | 0 | 6 | 4,2302 | 89. | 3 | 1 | 5 | 2,5589 | 11. | 2 | 4 | 4 | 2,0089 | 20. |
| 0 | 2 | 3 | 4,1422 | 312. | 3 | 2 | 2 | 2,5536 | 23. | 4 | 1 | 6 | 2,0009 | 10. |
| 2 | 1 | 1 | 4,1403 | 21. | 0 | 0 | 10 | 2,5381 | 9. | 0 | 3 | 10 | 1,9806 | 9. |
| 2 | 0 | 3 | 4,0935 | 18. | 0 | 3 | 6 | 2,5352 | 9. | 2 | 4 | 5 | 1,9545 | 8. |
| 1 | 1 | 5 | 4,0385 | 175. | 2 | 1 | 8 | 2,5308 | 9. | 3 | 1 | 10 | 1,9275 | 10. |
| 2 | 1 | 2 | 3,9843 | 44. | 2 | 3 | 3 | 2,5049 | 9. | 2 | 2 | 11 | 1,8971 | 17. |
| 0 | 1 | 6 | 3,8644 | 23. | 1 | 3 | 6 | 2,4470 | 10. | 4 | 3 | 1 | 1,8762 | 15. |
| 1 | 0 | 6 | 3,8544 | 171. | 2 | 3 | 4 | 2,4236 | 8. | 1 | 1 | 13 | 1,8737 | 12. |
| 0 | 2 | 4 | 3,8028 | 107. | 0 | 4 | 1 | 2,3649 | 12. | 3 | 4 | 2 | 1,8689 | 13. |
| 1 | 2 | 3 | 3,7875 | 45. | 3 | 0 | 7 | 2,3642 | 9. | 0 | 3 | 11 | 1,8649 | 9. |
| 2 | 0 | 4 | 3,7651 | 315. | 1 | 2 | 9 | 2,3474 | 12. | 1 | 5 | 2 | 1,8424 | 17. |
| 2 | 1 | 3 | 3,7594 | 176. | 2 | 3 | 5 | 2,3299 | 26. | 2 | 4 | 7 | 1,8287 | 10. |
| 1 | 1 | 6 | 3,5717 | 47. | 4 | 0 | 1 | 2,3289 | 10. | 5 | 1 | 2 | 1,8168 | 9. |
| 1 | 2 | 4 | 3,5229 | 31. | 3 | 2 | 5 | 2,3190 | 8. | 0 | 0 | 14 | 1,8129 | 31. |
| 2 | 1 | 4 | 3,5002 | 128. | 1 | 3 | 7 | 2,3113 | 10. | 5 | 1 | 3 | 1,7940 | 10. |
| 2 | 0 | 5 | 3,4398 | 34. | 3 | 1 | 7 | 2,2943 | 33. | 2 | 2 | 12 | 1,7858 | 9. |
| 0 | 1 | 7 | 3,3876 | 1000. | 1 | 4 | 1 | 2,2928 | 10. | 1 | 4 | 9 | 1,7834 | 16. |
| 1 | 0 | 7 | 3,3808 | 25. | 4 | 1 | 1 | 2,2619 | 12. | 4 | 3 | 5 | 1,7641 | 14. |
| 2 | 2 | 1 | 3,3046 | 159. | 0 | 3 | 8 | 2,2414 | 14. | 4 | 2 | 8 | 1,7501 | 9. |
| 1 | 2 | 5 | 3,2522 | 54. | 0 | 2 | 10 | 2,2386 | 9. | 1 | 1 | 14 | 1,7494 | 17. |
| 2 | 1 | 5 | 3,2344 | 10. | 2 | 0 | 10 | 2,2308 | 14. | 1 | 3 | 12 | 1,7286 | 20. |
| 2 | 2 | 2 | 3,2237 | 54. | 2 | 3 | 6 | 2,2289 | 10. | 2 | 5 | 3 | 1,7236 | 9. |
| 1 | 1 | 7 | 3,1852 | 32. | 3 | 3 | 0 | 2,2220 | 26. | 2 | 2 | 13 | 1,6846 | 10. |
| 0 | 0 | 8 | 3,1726 | 162. | 3 | 2 | 6 | 2,2193 | 36. | 5 | 1 | 6 | 1,6840 | 13 |
| 0 | 2 | 6 | 3,1592 | 77. | 3 | 3 | 1 | 2,2135 | 10. | 0 | 1 | 15 | 1,6659 | 12. |
| 2 | 2 | 3 | 3,1010 | 119. | 4 | 0 | 4 | 2,1944 | 29. | 3 | 5 | 2 | 1,6096 | 8. |
| 0 | 3 | 2 | 3,0728 | 39. | 3 | 3 | 2 | 2,1887 | 11. | 1 | 6 | 1 | 1,5583 | 9. |

2. Polymorphe selon la revendication 1 **caractérisé par** un thermogramme d'analyse calorimétrique différentielle à balayage (DSC) ayant un pic endothermique à 171,5 °C.

3. Procédé de préparation du polymorphe selon la revendication 1, comprenant l'échauffement de la forme solvatée du mononitrate d'arasertaconazole avec ½ mole d'acétone, de formule (IV): 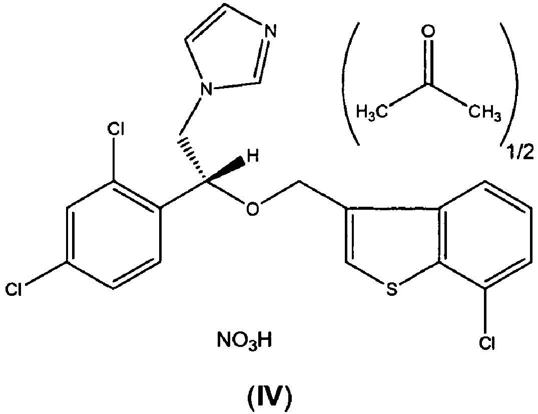 à une température de 354,2 °K.

4. Procédé de préparation du polymorphe selon la revendication 1, comprenant l'échauffement du polymorphe I de ladite composition à 373 °K sur une période de 2 à 24 h.

5. Procédé de préparation du polymorphe selon la revendication 1, comprenant la cristallisation du polymorphe I ou de la forme solvatée du mononitrate d'arasertaconazole avec ½ mole d'acétone (IV) dans de l'eau.

6. Utilisation du polymorphe selon la revendication 1 pour la préparation d'une composition pharmaceutique pour le traitement ou prévention d'infections de la peau et de la muqueuse provoquées par des champignons et levures chez les humains ou chez les animaux domestiques.

7. Polymorphe selon la revendication 1 destiné à être utilisé pour le traitement ou prévention d'infections de la peau ou de la membrane muqueuse provoquées par des champignons et des levures.

8. Utilisation du polymorphe selon la revendication 1 pour le traitement ou prévention de maladies des cultures provoquées par des champignons et levures.

9. Composition pharmaceutique comprenant le polymorphe selon la revendication 1 et des supports acceptables sur le plan pharmaceutique destinée à être utilisée pour le traitement ou prévention d'infections de la peau ou muqueuse provoquées par des champignons et levures chez les humains ou chez les animaux domestiques.

10. Composition agrichole comprenant le polymorphe selon la revendication 1 et des supports acceptables sur le plan pharmaceutique pour le traitement ou prévention de maladies de cultures provoquées par des champignons et levures.
